# EUROPEAN PATENT APPLICATION

(11) **EP 4 803 023 A1**
(43) Date of publication of application: **09.09.2026**
(21) Application number: 26160483.9
(22) Date of filing: 24.02.2026
(51) Int. Cl.: A61B 34/00

(54) **ROBOTIC SURGERY SYSTEM WITH CUTTING TOOL SELECTION BASED ON THE PLANNED ORIENTATION OF AN IMPLANT HAVING A CURVED SIDE AND A FLAT SIDE.**

(30) Priority: 07.03.2025 US 202563768421 P
(71) Applicant: Mako Surgical Corp., Weston, FL 33331 (US)
(72) Inventor: THOMPSON, Matthew, Durham, 27707 (US); CHANDRA, Varun, Fort Lauderdale, 33314 (US)
(74) Representative: Gill Jennings & Every LLP

(57) **Abstract**

A surgical robotics system includes a robotic arm, a first cutting tool, a second cutting tool, and a computer. The computer is programmed to obtain a planned pose of an implant augment relative to a bone, the implant augment having a curved side and a flat side, determine whether the curved side or the flat side faces the bone, in response to a determination that the curved side faces the bone, control the robotic arm to guide the first cutting tool to prepare the bone to receive the implant augment, and in response to a determination that the flat side faces the bone, control the robotic arm to guide the second cutting tool to prepare the bone to receive the implant augment.

## Description

### CROSS-REFERENCE TO RELATED APPLICATION

This application claims the benefit of and priority to U.S. Provisional Application No. 63/768421 filed March 7, 2025.

### BACKGROUND

The present disclosure relates generally to surgical systems for orthopedic surgeries, and more particularly to surgical systems for total and partial hip arthroplasty procedures. Hip arthroplasty, colloquially referred to as hip replacement, is widely used to treat hip osteoarthritis and other damage to a patient's hip joint by replacing portions of the hip anatomy with prosthetic components.

One possible tool for use in total hip arthroplasty procedure is a robotically-assisted surgical system. A robotically-assisted surgical system typically includes a robotic device that is used to prepare a patient's anatomy, a tracking system configured to monitor the location of the robotic device relative to the patient's anatomy, and a computing system configured to monitor and control the robotic device. Robotically-assisted surgical systems, in various forms, autonomously carry out surgical tasks, provide force feedback to a user manipulating a surgical device to complete surgical tasks, augment surgeon dexterity and precision, and/or provide other navigational cues to facilitate safe and accurate surgical operations.

A surgical plan is typically established prior to performing a surgical procedure with a robotically-assisted surgical system. Based on the surgical plan, the surgical system guides, controls, or limits movements of the surgical tool during portions of the surgical procedure. Guidance and/or control of the surgical tool serves to protect the patient and to assist the surgeon during implementation of the surgical plan.

### SUMMARY

In various embodiments, a surgical robotics system includes a robotic arm, a first cutting tool, a second cutting tool, and a computer. The computer is programmed to obtain a planned pose of an implant augment relative to a bone, the implant augment having a curved side and a flat side, determine whether the curved side or the flat side faces the bone, in response to a determination that the curved side faces the bone, control the robotic arm to guide the first cutting tool to prepare the bone to receive the implant augment, and in response to a determination that the flat side faces the bone, control the robotic arm to guide the second cutting tool to prepare the bone to receive the implant augment.

The second cutting tool may be a planer. The computer may be programmed to control the robotic arm to guide the guide the second cutting tool by defining a virtual cylinder aligned with the planned pose and controlling the robotic arm to constrain the planer to within the virtual cylinder. The first cutting tool may be a reamer, the planer and the reamer may be selectively attachable to a shaft held by the robotic arm, and the surgical robotics system may further comprise a power tool operate to provide torque via the shaft to the planer when the planer is attached to the shaft and to the reamer when the reamer is attached to the shaft.

The computer may be further programmed to control, in response to the determination that the flat side faces the bone, the robotic arm to guide the first cutting tool to prepare the bone to receive an acetabular cup in a reaming stage. The computer may be further programmed to instruct, in response to completion of the reaming stage, a user to detach the first cutting tool from the robotic arm and attach the second cutting tool to the robotic arm.

The computer may be programmed to determine whether the curved side or the flat side faces the bone by ascertaining an angle of the implant augment in the planned pose relative to a coordinate system defined based on landmarks of the bone and comparing the angle to a range or threshold.

Alternatively, or additionally, the computer may be programmed to determine whether the curved side or the flat side faces the bone by projecting a ray from the flat side and checking whether the ray intersects a virtual model of the bone.

The computer may be programmed to obtain the planned pose of the implant augment relative to the bone by providing a virtual environment comprising virtual models of the implant augment, the bone, and an implant and enabling a user to adjust relative positions of the virtual models. The computer may be programmed to enable the user to adjust relative positions of the virtual models by rotating a virtual acetabular cup model about a central axis thereof. The computer may be further programmed to enable placement of virtual screws in the virtual environment. The surgical robotics system may comprise a display screen. The computer may be programmed to cause the virtual environment to be presented on the display screen via a graphical user interface, wherein the graphical user interface further comprises a button selectable to flip over the virtual model of the implant augment.

In various embodiments, a method of configuring a surgical robotics system includes obtaining a planned pose of an implant augment relative to a bone, the implant augment having a curved side and a flat side, automatically making a determination as to whether the curved side or the flat side faces the bone, and automatically selecting between cutting tools based on the determination as to whether the curved side or the flat side faces the bone. Optionally, the method may include controlling a robotic arm to guide the selected cutting tool in preparing the bone to receive the implant augment. However, it will be appreciated that the surgical step is not strictly necessary provided that an appropriate cutting tool is selected. Thus, the method may be a non-surgical method. At least one of the steps of the method may be computer-implemented and/or automated and/or performed automatically.

The method may include coupling (in response to the determination) the selected cutting tool to the robotic arm. The method may include determining one or more control instructions for controlling a robotic arm to guide the selected cutting tool in preparing the bone to receive the implant augment.

The selected cutting tool may be a planar. Where the method includes controlling the robotic arm to guide the planer in preparing the bone to receive the implant augment, this step may comprise constraining, by the robotic arm, the planer to a line or cylinder.

The cutting tools may comprise a reamer and a planer. In response to the determination being that the flat side faces the bone, a planar may be automatically selected as the selected cutting tool. In response to the determination being that the curved side faces the bone, a reamer may be automatically selected as the selected cutting tool. The method may comprise detaching the reamer from the robotic arm and attaching the planer to the robotic arm in response to selecting the planer based on the determination being that the flat side faces the bone. In response to the determination being that the curved side faces the bone, the method may include selecting the reamer as the cutting tool. The reamer may then be used to ream the bone.

The step of automatically making the determination as to whether the curved side or the flat side faces the bone may comprise: ascertaining, by a computer, an angle of the implant augment in the planned pose in a coordinate system, the coordinate system based on landmarks of the bone; and comparing, by the computer, the angle to a range or threshold.

The step of obtaining the planned pose of the implant augment relative to the bone may comprise providing a graphical user interface enabling a user to adjust the planned pose by moving a virtual augment model relative to a virtual bone model. Optionally, moving the virtual augment model may comprise flipping the virtual augment model upside down.

The step of automatically selecting between cutting tools comprises selecting a burr as the selected cutting tool in response to determining that the flat side faces the bone.

### BRIEF DESCRIPTION OF THE DRAWINGS

FIG. 1A is a perspective view of a femur and a pelvis.
FIG. 1B is a perspective view of a hip joint formed by the femur and pelvis of FIG. 1A.
FIG. 1C is an exploded perspective view of a femoral component and an acetabular component for a total hip replacement procedure, according to some embodiments.
FIG. 1D is a perspective view illustrating placement of the femoral component and acetabular component of FIG. 1C in relation to the femur and pelvis of FIG. 1A, respectively, according to some scenarios.
FIG. 2 is an illustration of a surgical system, according to some embodiments.
FIG. 3 is a flowchart of a process for facilitating an arthroplasty procedure, according to some embodiments.
FIG. 4 is a first illustration of a graphical user interface that can be used with the process of FIG. 3, according to some embodiments.
FIG. 5 is a second illustration of a graphical user interface that can be used with the process of FIG. 3, according to some embodiments.
FIG. 6 is a third illustration of a graphical user interface that can be used with the process of FIG. 3, according to some embodiments.
FIG. 7 is a fourth illustration of a graphical user interface that can be used with the process of FIG. 3, according to some embodiments.
FIG. 8 is a flowchart of a process for facilitating implant clocking and screw planning during the process of FIG. 3, according to some embodiments.
FIG. 9 is a fifth illustration of a graphical user interface that can be used with the process of FIG. 3, according to some embodiments.
FIG. 10 is a flowchart of a process for determining screw lengths for screws used during the process of FIG. 8, according to some embodiments.
FIG. 11 is a sixth illustration of a graphical user interface that can be used with the process of FIG. 3, according to some embodiments.
FIG. 12 is a flowchart of showing a detailed view of steps of the process of FIG. 3, according to some embodiments.
FIG. 13 is a first visualization of registration regions on a pelvis for use with the process of FIG. 3, according to some embodiments.
FIG. 14 is a second visualization of registration regions on a pelvis for use with the process of FIG. 3, according to some embodiments.
FIG. 15 is a seventh illustration of a graphical user interface that can be used with the process of FIG. 3, according to some embodiments.
FIG. 16 is an eighth illustration of a graphical user interface that can be used with the process of FIG. 3, according to some embodiments.
FIG. 17 is a depiction of an implant augment and a probe as used in the process of FIG. 3, according to some embodiments.
FIG. 18 is a depiction of fixation of the implant augment of FIG. 17 to a bone as in the process of FIG. 3, according to some embodiments.
FIG. 19 is a flowchart of a process for clocking the implant during the process of FIG. 8, according to some embodiments.
FIG. 20 is a ninth illustration of a graphical user interface that can be used with the process of FIG. 3, according to some embodiments.
FIG. 21 is a tenth illustration of a graphical user interface that can be used with the process of FIG. 3, according to some embodiments.
FIG. 22 is a depiction of a cup impaction step of the process of FIG. 3, according to some embodiments.
FIG. 23 is a depiction of a cement curing step of the process of FIG. 3, according to an some embodiments.
FIG. 24 is an eleventh illustration of a graphical user interface that can be used with the process of FIG. 3, according to some embodiments.
FIG 25 is a flowchart of a process for automatically selecting an inverted augment mode and preparing a bone to receive an inverted augment, according to some embodiments.
FIG 26 is an illustration of a surgical system which can be used together with the process of FIG. 25, according to some embodiments.

### DETAILED DESCRIPTION

Presently preferred embodiments of the invention are illustrated in the drawings. An effort has been made to use the same or like reference numbers throughout the drawings to refer to the same or like parts. Although this specification refers primarily to a robotic arm for orthopedic hip replacement, it should be understood that the subject matter described herein is applicable to other types of robotic systems, including those used for surgical and non-surgical applications, as well as to other joints of the body, such as, for example, a knee or shoulder joint.

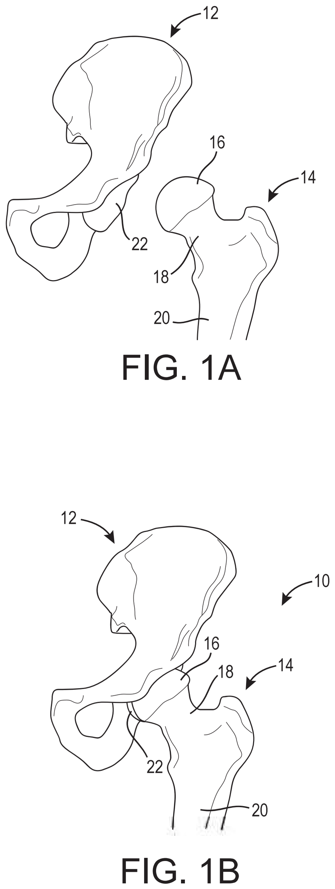

The hip joint is the joint between the femur and the pelvis and primarily functions to support the weight of the body in static (for example, standing) and dynamic (for example, walking) postures. FIG. 1A illustrates the bones of a hip joint 10, which include a pelvis 12 (shown in part) and a proximal end of a femur 14. The proximal end of the femur 14 includes a femoral head 16 disposed on a femoral neck 18. The femoral neck 18 connects the femoral head 16 to a femoral shaft 20. As shown in FIG. 1B, the femoral head 16 fits into a concave socket in the pelvis 12 called the acetabulum 22, thereby forming the hip joint 10. The acetabulum 22 and femoral head 16 are both covered by articular cartilage that absorbs shock and promotes articulation of the hip joint 10.

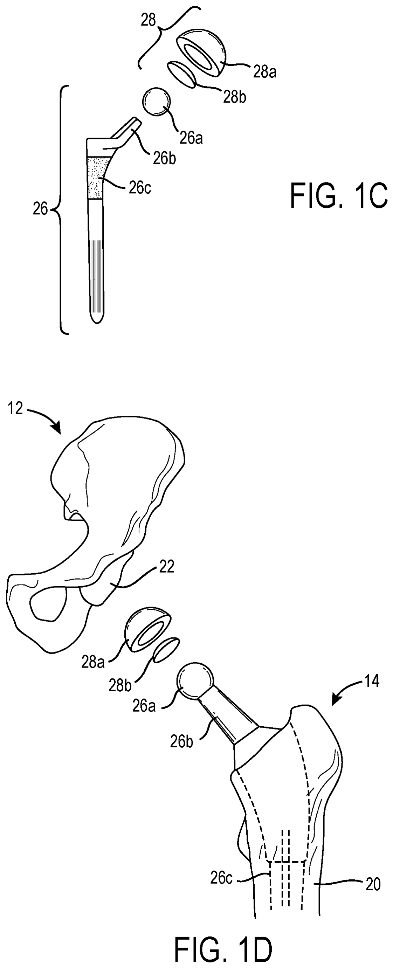

Over time, the hip joint 10 may degenerate (for example, due to osteoarthritis) resulting in pain and diminished functionality. As a result, a hip replacement procedure, such as total hip arthroplasty or hip resurfacing, may be necessary. During hip replacement, a surgeon replaces portions of a patient's hip joint 10 with artificial components. In total hip arthroplasty, the surgeon removes the femoral head 16 and the femoral neck 18 and replaces the natural bone with a prosthetic femoral component 26 comprising a head 26a, a neck 26b, and a stem 26c (shown in FIG. 1C). As shown in FIG. 1D, the stem 26c of the prosthetic femoral component 26 is anchored in a cavity the surgeon creates in the intramedullary canal of the femur 14. Alternatively, if disease is confined to the surface of the femoral head 16, the surgeon may opt for a less invasive approach in which the femoral head 16 is resurfaced (e.g., using a cylindrical reamer) and then mated with a prosthetic femoral head cup (not shown).

Similarly, if the natural acetabulum 22 of the pelvis 12 is worn or diseased, the surgeon resurfaces the acetabulum 22 using a reamer and replaces the natural surface with a prosthetic acetabular component 28 comprising a hemispherical shaped cup 28a (shown in FIG. 1C) that may include a liner 28b. To install the prosthetic acetabular component 28, the surgeon connects the cup 28a to a distal end of an impactor tool and implants the cup 28a into the reamed acetabulum 22 by repeatedly striking a proximal end of the impactor tool with a mallet. If the acetabular component 28 includes a liner 28b, the surgeon snaps the liner 28b into the cup 28a after implanting the cup 28a. Depending on the position in which the surgeon places the patient for surgery, the surgeon may use a straight or offset reamer to ream the acetabulum 22 and a straight or offset impactor to implant the cup 28a. For example, a surgeon that uses a postero-lateral approach may prefer straight reaming and impaction whereas a surgeon that uses an antero-lateral approach may prefer offset reaming and impaction.

In some cases, an implant augment is used to support or otherwise facilitate reconstruction of the acetabulum 22 to facilitate fixation of the cup 28a to the pelvis 12 in a preferred position and orientation. Use of an augment may be preferable in several scenarios. As one example, an implant augment may be advantageous post-traumatic hip reconstructions, in which a traumatic injury (e.g., car crash, etc.) caused damage to the pelvis 12. As another example, an implant augment may be advantageous in cases of hip dysplasia or other cases of acetabular bone loss, i.e., to fill space created by such bone loss. As another example, an implant augment may be advantageous for revision hip arthroplasty procedures, in which a previously-implanted hip prosthesis is removed and replaced with a new implant due to degradation of neighboring bone or other complications.

Current surgical procedures that involve implant augments typically rely on surgeon expertise and experience to manually place an implant augment in a position that looks and feels correct to the surgeon intraoperatively. Such procedures may be difficult and result in extended surgical time. Additionally, currently-available robotically-assisted surgical devices for hip arthroplasty do not provide for placement of implant augments. The systems and methods described herein provide for computer-assisted planning of implant placement and robotically-assisted surgical steps to facilitate bone preparation for implant augments and placement of implant augments during hip arthroplasty procedures, thereby facilitating hip arthroplasty procedures in cases of bone loss, traumatic injury, revision hip replacements, or other relevant scenarios. The systems and methods described herein may thereby improve patient outcomes, reduce surgery times, and reduce the burden on surgeons for augmented hip arthroplasty procedures.

One implementation of the present disclosure is a method. The method includes displaying, on a graphical user interface, a planned position of a virtual implant model relative to a virtual bone model of a bone, where the virtual implant model includes a plurality of virtual screw holes offset from a central axis of the virtual implant model; determining a planned rotational orientation of the virtual implant model by rotating, on the graphical user interface, the virtual implant model about the central axis of the virtual implant model such that the plurality of virtual screw holes rotate about the central axis; controlling a robotic device to guide preparation of the bone to receive a physical implant in the planned position; and providing computer-assisted navigation configured to guide the physical implant into physical rotational alignment with the planned rotational orientation of the virtual implant model.

Another implementation of the present disclosure is a system. The system includes a robotic device and a circuitry. The circuitry is configured to display, on a graphical user interface, a planned position of a virtual implant model relative to a virtual bone model of a bone, where the virtual implant model includes a plurality of virtual screw holes offset from a central axis of the virtual implant model; determine a planned rotational orientation of the virtual implant model by rotating, on the graphical user interface, the virtual implant model about the central axis of the virtual implant model such that the plurality of virtual screw holes rotate about the central axis; control a robotic device to guide preparation of the bone to receive a physical implant in the planned position; and provide computer-assisted navigation configured to guide the physical implant into physical rotational alignment with the planned rotational orientation of the virtual implant model.

Another implementation of the present disclosure relates to one or more non-transitory computer-readable media storing instructions that, when executed by a processor, cause the processor to perform operations. The operations include displaying, on a graphical user interface, a planned position of a virtual implant model relative to a virtual bone model of a bone, where the virtual implant model includes a plurality of virtual screw holes offset from a central axis of the virtual implant model; determining a planned rotational orientation of the virtual implant model by rotating, on the graphical user interface, the virtual implant model about the central axis of the virtual implant model such that the plurality of virtual screw holes rotate about the central axis; controlling a robotic device to guide preparation of the bone to receive a physical implant in the planned position; and providing computer-assisted navigation configured to guide the physical implant into physical rotational alignment with the planned rotational orientation of the virtual implant model.

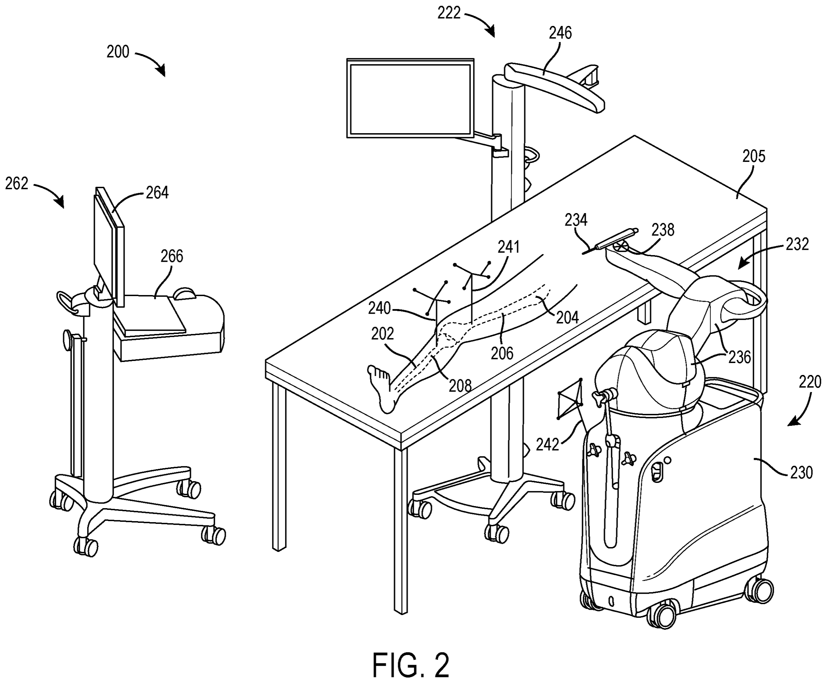

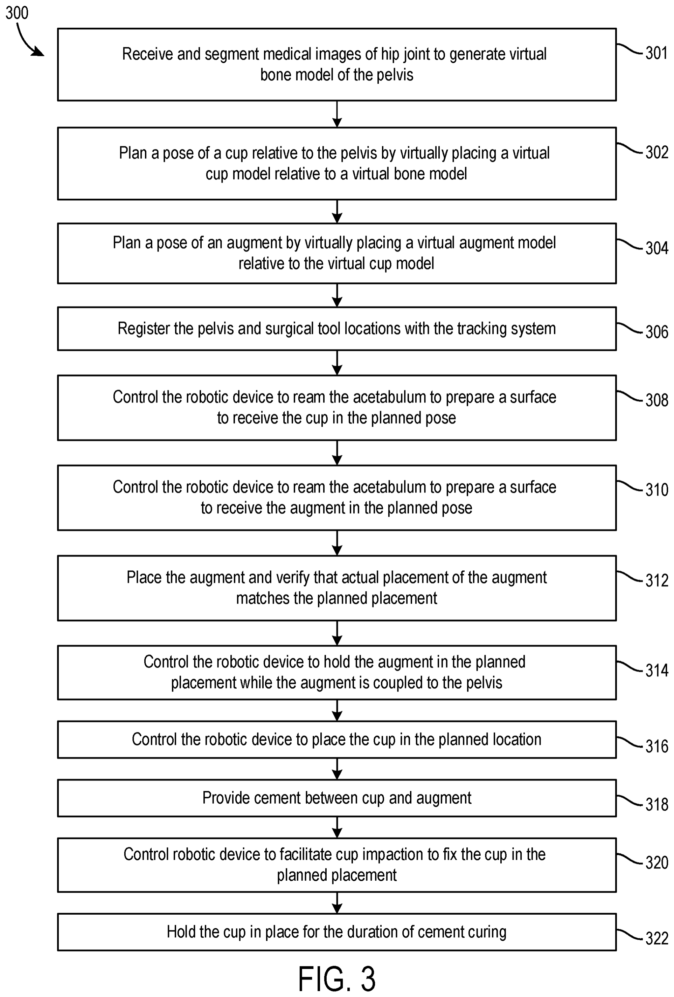

Referring now to FIG. 2, a surgical system 200 for orthopedic surgery is shown, according to an exemplary embodiment. In general, the surgical system 200 is configured to facilitate the planning and execution of a surgical plan, for example to facilitate a joint-related procedure. As shown in FIG. 2, the surgical system 200 is set up to treat a leg 202 of a patient 204 sitting or lying on table 205. In the illustration shown in FIG. 2, the leg 202 includes femur 206 and tibia 208, between which a prosthetic knee implant is to be implanted in a total knee arthroscopy procedure. In other scenarios, for example as described herein with reference to 1A-1D and FIGS. 3-23, the surgical system 200 is set up to treat the hip joint 10 of a patient, i.e., the femur 14 and the pelvis 12 of the patient (illustrated in FIGS. 1A-1D). Additionally, in still other scenarios, the surgical system 200 is set up to treat a shoulder of a patient, i.e., to facilitate replacement and/or augmentation of components of a shoulder joint (e.g., to facilitate placement of a humeral component, a glenoid component, and a graft or implant augment). Various other anatomical regions and procedures are also possible. To facilitate the procedure, surgical system 200 includes robotic device 220, tracking system 222, and computing system 224.

The robotic device 220 is configured to modify a patient's anatomy (e.g., femur 206 of patient 204) under the control of the computing system 224. One embodiment of the robotic device 220 is a haptic device. "Haptic" refers to a sense of touch, and the field of haptics relates to, among other things, human interactive devices that provide feedback to an operator. Feedback may include tactile sensations such as, for example, vibration. Feedback may also include providing force to a user, such as a positive force or a resistance to movement. One use of haptics is to provide a user of the device with guidance or limits for manipulation of that device. For example, a haptic device may be coupled to a surgical tool, which can be manipulated by a surgeon to perform a surgical procedure. The surgeon's manipulation of the surgical tool can be guided or limited through the use of haptics to provide feedback to the surgeon during manipulation of the surgical tool.

Another embodiment of the robotic device 220 is an autonomous or semi-autonomous robot. "Autonomous" refers to a robotic device's ability to act independently or semi-independently of human control by gathering information about its situation, determining a course of action, and automatically carrying out that course of action. For example, in such an embodiment, the robotic device 220, in communication with the tracking system 222 and the computing system 224, may autonomously complete the series of femoral cuts mentioned above without direct human intervention.

The robotic device 220 includes a base 230, a robotic arm 232, and a surgical tool 234, and is communicably coupled to the computing system 224 and the tracking system 222. The base 230 provides a moveable foundation for the robotic arm 232, allowing the robotic arm 232 and the surgical tool 234 to be repositioned as needed relative to the patient 204 and the table 205. The base 230 may also contain power systems, computing elements, motors, and other electronic or mechanical system necessary for the functions of the robotic arm 232 and the surgical tool 234 described below.

The robotic arm 232 is configured to support the surgical tool 234 and provide a force as instructed by the computing system 224. In some embodiments, the robotic arm 232 allows a user to manipulate the surgical tool 234 and provides force feedback to the user. In such an embodiment, the robotic arm 232 includes joints 236 and mount 238 that include motors, actuators, or other mechanisms configured to allow a user to freely translate and rotate the robotic arm 232 and surgical tool 234 through allowable poses while providing force feedback to constrain or prevent some movements of the robotic arm 232 and surgical tool 234 as instructed by computing system 224. As described in detail below, the robotic arm 232 thereby allows a surgeon to have full control over the surgical tool 234 within a control object while providing force feedback along a boundary of that object (e.g., a vibration, a force preventing or resisting penetration of the boundary). In some embodiments, the robotic arm 232 is configured to move the surgical tool 234 to a new pose automatically without direct user manipulation, as instructed by computing system 224, in order to position the robotic arm 232 as needed and/or complete certain surgical tasks, including, for example, cuts in a femur 206 or an acetabulum.

The surgical tool 234 is configured to cut, burr, grind, drill, partially resect, reshape, and/or otherwise modify a bone. The surgical tool 234 may be any suitable tool, and may be one of multiple tools interchangeably connectable to robotic device 220. For example, as shown in FIG. 2 the surgical tool 234 is a spherical burr. The surgical tool 234 may also be a sagittal saw, for example with a blade aligned parallel with a tool axis or perpendicular to the tool axis. The surgical tool 234 may also be a holding arm or other support configured to hold an implant component (e.g., cup 28a, implant augment, etc.) in position while the implant component is screwed to a bone, adhered (e.g., cemented) to a bone or other implant component, or otherwise installed in a preferred position. In some embodiments, the surgical tool 234 is an impaction tool configured to provide an impaction force to a cup 28a to facilitate fixation of the cup 28a to a pelvis 12 in a planned location and orientation.

Tracking system 222 is configured to track the patient's anatomy (e.g., femur 206 and tibia 208) and the robotic device 220 (i.e., surgical tool 234 and/or robotic arm 232) to enable control of the surgical tool 234 coupled to the robotic arm 232, to determine a position and orientation of modifications or other results made by the surgical tool 234, and allow a user to visualize the bones (e.g., femur 206, the tibia 208, pelvis 12, humerus, scapula, etc. as applicable in various procedures), the surgical tool 234, and/or the robotic arm 232 on a display of the computing system 224. More particularly, the tracking system 222 determines a position and orientation (i.e., pose) of objects (e.g., surgical tool 234, femur 206) with respect to a coordinate frame of reference and tracks (i.e., continuously determines) the pose of the objects during a surgical procedure. According to various embodiments, the tracking system 222 may be any type of navigation system, including a non-mechanical tracking system (e.g., an optical tracking system), a mechanical tracking system (e.g., tracking based on measuring the relative angles of joints 236 of the robotic arm 232), or any combination of non-mechanical and mechanical tracking systems.

In the embodiment shown in FIG. 2, the tracking system 222 includes an optical tracking system. Accordingly, tracking system 222 includes a first fiducial tree 240 coupled to the tibia 208, a second fiducial tree 241 coupled to the femur 206, a third fiducial tree 242 coupled to the base 230, one or more fiducials coupled to surgical tool 234, and a detection device 246 configured to detect the three-dimensional position of fiducials (i.e., markers on fiducial trees 240-242). Fiducial trees 240, 241 may be coupled to other bones as suitable for various procedures (e.g., pelvis 12 and femur 206 in a hip arthroplasty procedure). Detection device 246 may be an optical detector such as a camera or infrared sensor. The fiducial trees 240-242 include fiducials, which are markers configured to show up clearly to the optical detector and/or be easily detectable by an image processing system using data from the optical detector, for example by being highly reflective of infrared radiation (e.g., emitted by an element of tracking system 222). A stereoscopic arrangement of cameras on detection device 246 allows the position of each fiducial to be determined in 3D-space through a triangulation approach. Each fiducial has a geometric relationship to a corresponding object, such that tracking of the fiducials allows for the tracking of the object (e.g., tracking the second fiducial tree 241 allows the tracking system 222 to track the femur 206), and the tracking system 222 may be configured to carry out a registration process to determine or verify this geometric relationship. Unique arrangements of the fiducials in the fiducial trees 240-242 (i.e., the fiducials in the first fiducial tree 240 are arranged in a different geometry than fiducials in the second fiducial tree 241) allows for distinguishing the fiducial trees 240-242, and therefore the objects being tracked, from one another.

Using the tracking system 222 of FIG. 2 or some other approach to surgical navigation and tracking, the surgical system 200 can determine the position of the surgical tool 234 relative to a patient's anatomical feature, for example femur 206, as the surgical tool 234 is used to modify the anatomical feature or otherwise facilitate the surgical procedure. Additionally, using the tracking system 222 of FIG. 2 or some other approach to surgical navigation and tracking, the surgical system 200 can determine the relative poses of the tracked bones.

The computing system 224 is configured to create a surgical plan. The computing system 224 may be configured to control the robotic device 220 in accordance with the surgical plan to make one or more bone modifications and/or facilitate implantation of one or more prosthetic components. Accordingly, the computing system 224 is communicably coupled to the tracking system 222 and the robotic device 220 to facilitate electronic communication between the robotic device 220, the tracking system 222, and the computing system 224. Further, the computing system 224 may be connected to a network to receive information related to a patient's medical history or other patient profile information, medical imaging, surgical plans, surgical procedures, and to perform various functions related to performance of surgical procedures, for example by accessing an electronic health records system. Computing system 224 includes processing circuit 260 and input/output device 262.

The input/output device 262 is configured to receive user input and display output as needed for the functions and processes described herein. As shown in FIG. 2, input/output device 262 includes a display 264 and a keyboard 266. The display 264 is configured to display graphical user interfaces generated by the processing circuit 260 that include, for example, information about surgical plans, medical imaging, settings and other options for the surgical system 200, status information relating to the tracking system 222 and the robotic device 220, and tracking visualizations based on data supplied by the tracking system 222. The keyboard 266 is configured to receive user input to those graphical user interfaces to control one or more functions of the surgical system 200.

The processing circuit 260 includes a processor and memory device. The processor can be implemented as a general purpose processor, an application specific integrated circuit (ASIC), one or more field programmable gate arrays (FPGAs), a group of processing components, or other suitable electronic processing components. The memory device (e.g., memory, memory unit, storage device, etc.) is one or more devices (e.g., RAM, ROM, Flash memory, hard disk storage, etc.) for storing data and/or computer code for completing or facilitating the various processes and functions described in the present application. The memory device may be or include volatile memory or non-volatile memory. The memory device may include database components, object code components, script components, or any other type of information structure for supporting the various activities and information structures described in the present application. According to an exemplary embodiment, the memory device is communicably connected to the processor via the processing circuit 260 and includes computer code for executing (e.g., by the processing circuit 260 and/or processor) one or more processes described herein.

More particularly, processing circuit 260 is configured to facilitate the creation of a preoperative surgical plan prior to the surgical procedure. According to some embodiments, the preoperative surgical plan is developed utilizing a three-dimensional representation of a patient's anatomy, also referred to herein as a "virtual bone model." A "virtual bone model" may include virtual representations of cartilage or other tissue in addition to bone. To obtain the virtual bone model, the processing circuit 260 receives imaging data of the patient's anatomy on which the surgical procedure is to be performed (e.g., femur 206, pelvis 12). The imaging data may be created using any suitable medical imaging technique to image the relevant anatomical feature, including computed tomography (CT), magnetic resonance imaging (MRI), and/or ultrasound. The imaging data is then segmented (i.e., the regions in the imaging corresponding to different anatomical features are distinguished) to obtain the virtual bone model. For example, as described in further detail below, MRI-based scan data of a hip can be segmented to distinguish the femur from surrounding ligaments, cartilage, previously-implanted prosthetic components, and other tissue to obtain a three-dimensional model of the imaged hip.

Alternatively, the virtual bone model may be obtained by selecting a three-dimensional model from a database or library of bone models. In one embodiment, the user may use input/output device 262 to select an appropriate model. In another embodiment, the processing circuit 260 may execute stored instructions to select an appropriate model based on images or other information provided about the patient. The selected bone model(s) from the database can then be deformed based on specific patient characteristics, creating a virtual bone model for use in surgical planning and implementation as described herein.

A preoperative surgical plan can then be created based on the virtual bone model. The surgical plan may be automatically generated by the processing circuit 260, input by a user via input/output device 262, or some combination of the two (e.g., the processing circuit 260 limits some features of user-created plans, generates a plan that a user can modify, etc.). In some embodiments, as described in detail below, the surgical plan may be generated and/or modified based on distraction force measurements collected intraoperatively. In some embodiments, the surgical plan may be modified based on qualitative intra-operational assessment of implant fixation (i.e., loose or fixed) and/or intra-operative bone defect mapping after primary implant removal, for example as described in detail below.

The preoperative surgical plan includes the desired cuts, holes, surfaces, burrs, or other modifications to a patient's anatomy to be made using the surgical system 200. For example, for a total knee arthroscopy procedure, the preoperative plan may include the cuts necessary to form, on a femur, a distal surface, a posterior chamfer surface, a posterior surface, an anterior surface, and an anterior chamfer surface in relative orientations and positions suitable to be mated to corresponding surfaces of the prosthetic to be joined to the femur during the surgical procedure, as well as cuts necessary to form, on the tibia, surface(s) suitable to mate to the prosthetic to be joined to the tibia during the surgical procedure. As another example, in a hip arthroplasty procedure, the surgical plan may include the burr necessary to form one or more surfaces on the acetabular region of the pelvis 12 to receive a cup 28a and, in suitable cases, an implant augment. Accordingly, the processing circuit 260 may receive, access, and/or store a model of the prosthetic to facilitate the generation of surgical plans.

The processing circuit 260 is further configured to generate a control object for the robotic device 220 in accordance with the surgical plan. The control object may take various forms according to the various types of possible robotic devices (e.g., haptic, autonomous, etc.). For example, in some embodiments, the control object defines instructions for the robotic device to control the robotic device to move within the control object (i.e., to autonomously make one or more cuts of the surgical plan guided by feedback from the tracking system 222). In some embodiments, the control object includes a visualization of the surgical plan and the robotic device on the display 264 to facilitate surgical navigation and help guide a surgeon to follow the surgical plan (e.g., without active control or force feedback of the robotic device). In embodiments where the robotic device 220 is a haptic device, the control object may be a haptic object as described in the following paragraphs.

In an embodiment where the robotic device 220 is a haptic device, the processing circuit 260 is further configured to generate one or more haptic objects based on the preoperative surgical plan to assist the surgeon during implementation of the surgical plan by enabling constraint of the surgical tool 234 during the surgical procedure. A haptic object may be formed in one, two, or three dimensions. For example, a haptic object can be a line, a plane, or a three-dimensional volume. A haptic object may be curved with curved surfaces and/or have flat surfaces, and can be any shape, for example a funnel shape. Haptic objects can be created to represent a variety of desired outcomes for movement of the surgical tool 234 during the surgical procedure. One or more of the boundaries of a three-dimensional haptic object may represent one or more modifications, such as cuts, to be created on the surface of a bone. A planar haptic object may represent a modification, such as a cut, to be created on the surface of a bone. A curved haptic object may represent a resulting surface of a bone as modified to receive a cup 28a and/or implant augment.

In an embodiment where the robotic device 220 is a haptic device, the processing circuit 260 is further configured to generate a virtual tool representation of the surgical tool 234. The virtual tool includes one or more haptic interaction points (HIPs), which represent and are associated with locations on the physical surgical tool 234. In an embodiment in which the surgical tool 234 is a spherical burr (e.g., as shown in FIG. 2), a HIP may represent the center of the spherical burr. If the surgical tool 234 is an irregular shape, for example as for a sagittal saw, the virtual representation of the sagittal saw may include numerous HIPs. Using multiple HIPs to generate haptic forces (e.g. positive force feedback or resistance to movement) on a surgical tool is described in U.S. application Ser. No. 13/339,369, titled "System and Method for Providing Substantially Stable Haptics," filed Dec. 28, 2011. In one embodiment of the present invention, a virtual tool representing a sagittal saw includes eleven HIPs. As used herein, references to a "HIP" are deemed to also include references to "one or more HIPs." As described below, relationships between HIPs and haptic objects enable the surgical system 200 to constrain the surgical tool 234.

Prior to performance of the surgical procedure, the patient's anatomy (e.g., femur 206) is registered to the virtual bone model of the patient's anatomy by any known registration technique. One possible registration technique is point-based registration, as described in U.S. Pat. No. 8,010,180, titled "Haptic Guidance System and Method," granted Aug. 30, 2011. Alternatively, registration may be accomplished by 2D/3D registration utilizing a hand-held radiographic imaging device, as described in U.S. application Ser. No. 13/562,163, titled "Radiographic Imaging Device," filed Jul. 30, 2012. Registration also includes registration of the surgical tool 234 to a virtual tool representation of the surgical tool 234, so that the surgical system 200 can determine and monitor the pose of the surgical tool 234 relative to the patient (i.e., to femur 206). Registration allows for accurate navigation, control, and/or force feedback during the surgical procedure. Additional details relating to registration for hip arthroplasty procedures in some embodiments are described in detail below.

The processing circuit 260 is configured to monitor the virtual positions of the virtual tool representation, the virtual bone model, and the control object (e.g., virtual haptic objects) corresponding to the real-world positions of the patient's bone (e.g., femur 206), the surgical tool 234, and one or more lines, planes, or three-dimensional spaces defined by forces created by robotic device 220. For example, if the patient's anatomy moves during the surgical procedure as tracked by the tracking system 222, the processing circuit 260 correspondingly moves the virtual bone model. The virtual bone model therefore corresponds to, or is associated with, the patient's actual (i.e. physical) anatomy and the position and orientation of that anatomy in real/physical space. Similarly, any haptic objects, control objects, or other planned automated robotic device motions created during surgical planning that are linked to cuts, modifications, etc. to be made to that anatomy also move in correspondence with the patient's anatomy. In some embodiments, the surgical system 200 includes a clamp or brace to substantially immobilize the femur 206 to minimize the need to track and process motion of the femur 206.

For embodiments where the robotic device 220 is a haptic device, the surgical system 200 is configured to constrain the surgical tool 234 based on relationships between HIPs and haptic objects. That is, when the processing circuit 260 uses data supplied by tracking system 222 to detect that a user is manipulating the surgical tool 234 to bring a HIP in virtual contact with a haptic object, the processing circuit 260 generates a control signal to the robotic arm 232 to provide haptic feedback (e.g., a force, a vibration) to the user to communicate a constraint on the movement of the surgical tool 234. In general, the term "constrain," as used herein, is used to describe a tendency to restrict movement. However, the form of constraint imposed on the surgical tool 234 depends on the form of the relevant haptic object. A haptic object may be formed in any desirable shape or configuration. As noted above, three exemplary embodiments include a line, plane, or three-dimensional volume. In one embodiment, the surgical tool 234 is constrained because a HIP of surgical tool 234 is restricted to movement along a linear haptic object. In another embodiment, the haptic object is a three-dimensional volume and the surgical tool 234 may be constrained by substantially preventing movement of the HIP outside of the volume enclosed by the walls of the three-dimensional haptic object. In another embodiment, the surgical tool 234 is constrained because a planar haptic object substantially prevents movement of the HIP outside of the plane and outside of the boundaries of the planar haptic object. For example, the processing circuit 260 can establish a planar haptic object corresponding to a planned planar distal cut needed to create a distal surface on the femur 206 in order to confine the surgical tool 234 substantially to the plane needed to carry out the planned distal cut.

For embodiments where the robotic device 220 is an autonomous device, the surgical system 200 is configured to autonomously move and operate the surgical tool 234 in accordance with the control object. For example, the control object may define areas relative to the femur 206 for which a cut should be made. In such a case, one or more motors, actuators, and/or other mechanisms of the robotic arm 232 and the surgical tool 234 are controllable to cause the surgical tool 234 to move and operate as necessary within the control object to make a planned cut, for example using tracking data from the tracking system 222 to allow for closed-loop control.

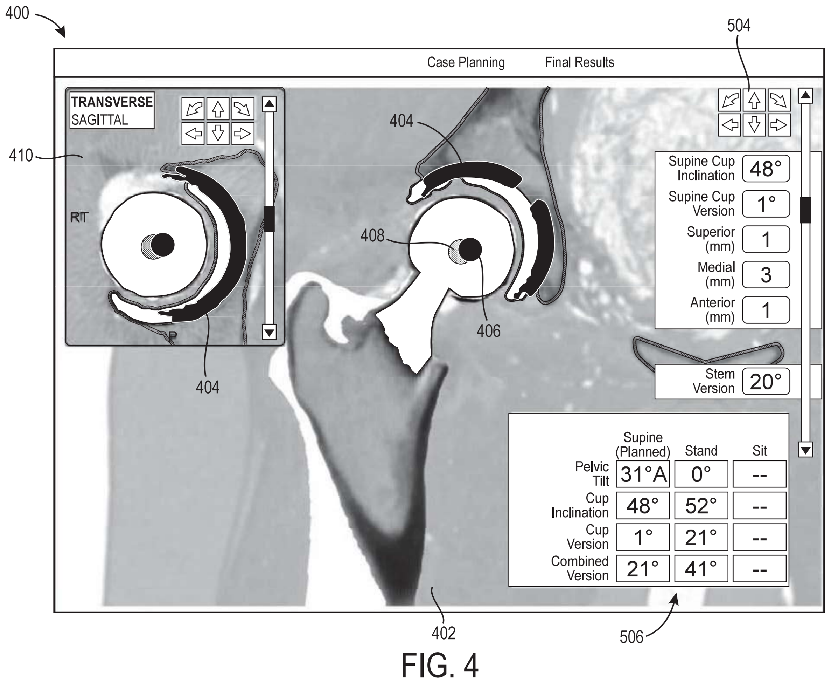

Referring now to FIG. 3, a flowchart of a process 300 for planning and conducting a hip arthroplasty procedure is shown, according to an exemplary embodiment. Process 300 can be executed by the surgical system 200 of FIG. 2. Additionally, FIGS. 4-23 show various systems, methods, graphical user interfaces, etc. used in process 300. Reference is made thereto to facilitate explanation of process 300. It should be understood that process 300 is not limited to the examples of FIGS. 4-23. Additionally, although FIGS. 3-23 illustrate embodiments of process 300 for planning and conducting a procedure relating to a hip, other embodiments are possible for planning and conducting procedures relating to other anatomy, for example shoulders or knees.

At step 301, medical images of the hip joint are received and segmented to generate a virtual bone model of the pelvis. For example, the medical images may be collected using CT technology, MRI technology, or some other medical imaging modality. The images are then segmented, i.e., processed to differentiate areas of the images that correspond to the pelvis, the femur, soft tissue, and/or one or more previously-implanted prosthetic components.

In revision hip arthroplasty cases (i.e., where a previously-implanted cup is shown in the images), a determination may be made of whether the previously-implanted cup is "fixed" (i.e., substantially rigidly coupled to the pelvis) or "loose" (i.e., at least partially detached from the pelvis"). If the previously-implanted cup is fixed, the shape, position, etc. of the previously-implanted cup may be determined and included in the virtual bone model of the pelvis, for example to facilitate registration at step 306 as described in detail below. If the previously-implanted cup is loose, the previously-implanted cup may be segmented out such that the loose cup is not included in the virtual bone model of the pelvis. Additionally, various corrections may be introduced to address distortions in CT or other imagery that may be caused by the materials of the previously-implanted cup and/or movement of a loose cup during imaging.

In some embodiments, step 301 is achieved automatically by the processing circuit 260 or other computing resource. In other embodiments, human input is used in cooperation with automated functions to achieve the segmentation and model generation of step 301.

At step 302, placement of an implant cup relative to the pelvis is planned by virtually placing a virtual cup model relative to a virtual bone model, i.e., relative to the virtual model of the pelvis generated at step 301 and, in some cases relative to previously-implanted components (e.g., primary cup, fracture plates, compression screws, etc.). The virtual cup model is a virtual representation of the cup implant to be implanted into the patient during the surgical procedure. Various cup sizes, shapes, types, etc. may be possible, and a different virtual cup model available for each cup. The virtual cup model is placed to provide a desired center of rotation for the hip joint (e.g., relative to the pelvis, relative to a patient's other hip, etc.) and ensure a full range of motion. Various software planning tools may be provided via the surgical system 200 to facilitate a surgeon or other user in selecting and evaluating the pose of the virtual cup model.

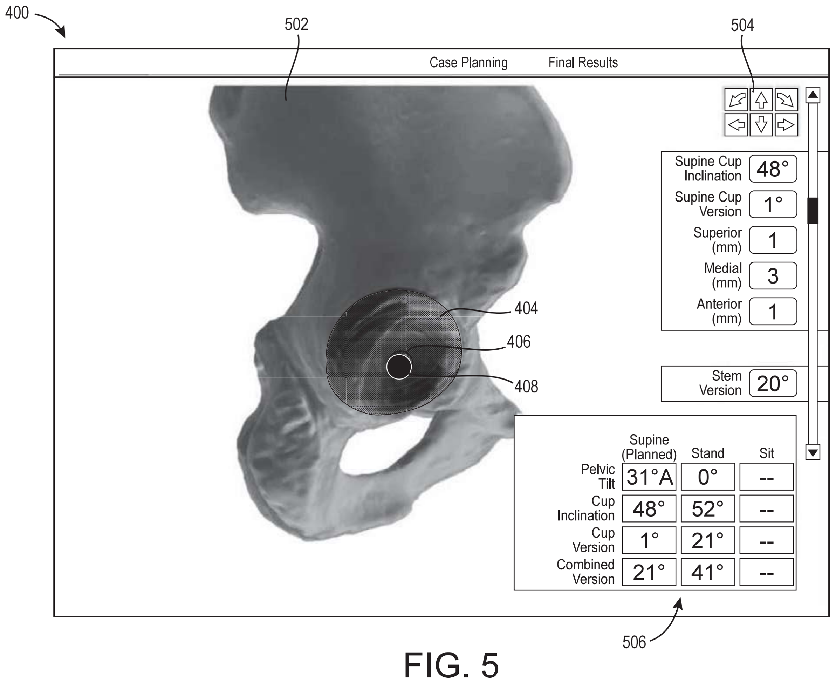

FIGS. 4-5 illustrate graphical user interfaces that can be generated by the processing circuit 260 and displayed on the display 264 to facilitate planning of cup placement at step 302. FIG. 4 shows a 2-dimensional visualization of a planned cup pose relative to CT images received at step 301. FIG. 5 shows a 3-dimensional visualization of the planned cup pose relative to a virtual bone model generated at step 301. Both are described in further detail below.

In FIG. 4, the graphical user interface 400 includes a first CT image 402 overlaid with a representation of the virtual implant cup 404. A center point (center of rotation) 406 of the virtual implant cup 404 is also shown. Additionally, as shown in FIG. 4, the graphical user interface 400 visualizes the previous center point 408 of the joint as imaged, i.e., before the surgical operation. In the example of FIG. 4, the graphical user interface 400 also shows a second CT image 410 (e.g., taken in a different plane) which is also overlaid with the virtual implant cup 404, the center point 406, and the previous center point 408. Advantageously, bone density information may be visible in the CT images 402, 410. The graphical user interface 400 may thereby facilitate a surgeon in determining placement of the virtual implant cup 404 relative to the imaged bones at step 302.

In FIG. 5, the graphical user interface 400 includes a 3-dimensional visualization of the virtual bone model 502 and of the virtual implant cup 404 placed relative to the virtual bone model 502. The graphical user interface 400 includes a previous center point 408 indicating a center of rotation of the hip joint as determined from the images as well as a center point 406 of the virtual implant cup 404. The graphical user interface 400 thereby facilitates a surgeon in viewing and adjusting the planned pose of the virtual implant cup 404.

As shown in FIGS. 4-5, the graphical user interface 400 includes control arrows 504 that can be selected to translate or rotate the virtual implant cup 404 relative to the virtual bone model 502. The graphical user interface 400 also includes data fields 506 that show various information that may be of interest to the user, for example, pelvic tilt, cup inclination, cup version, stem version, combined version, and superior, medial, and anterior distances. The graphical user interface 400 of FIGS. 4-5 thereby facilitates planning of implant cup placement relative to the pelvis at step 302.

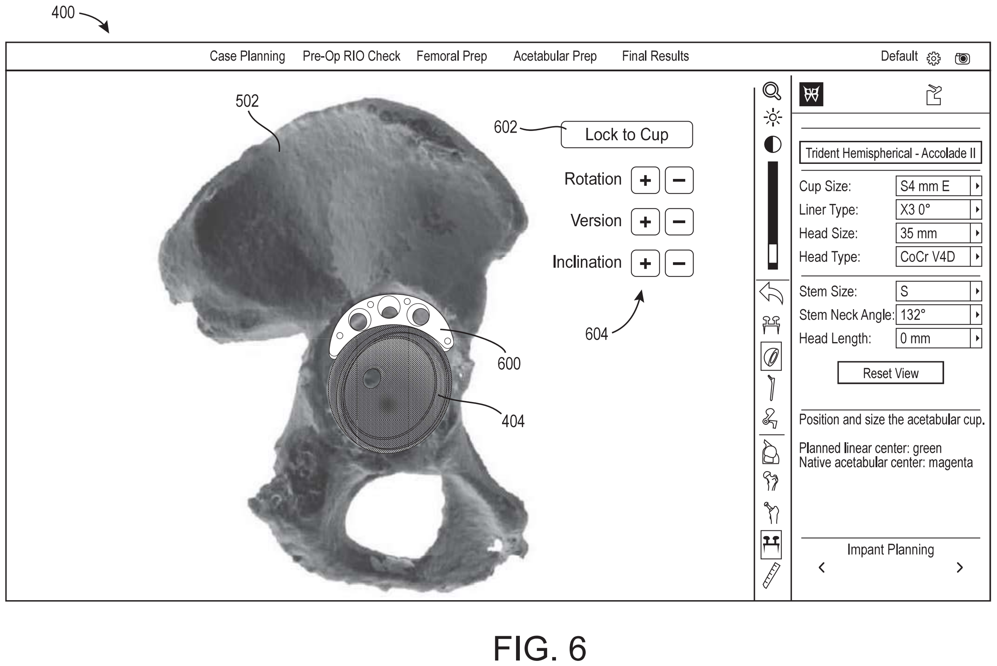

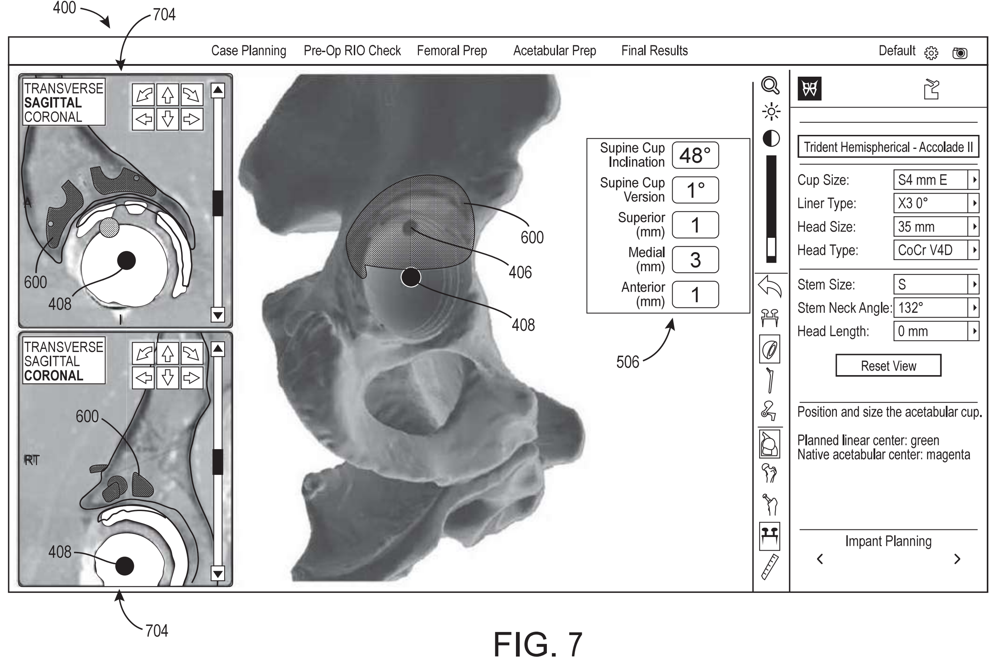

At step 304, placement of an implant augment is planned by virtually placing a virtual augment model relative to the virtual implant cup. For example, a determination may be made based on the visualization of the virtual bone model 502 of FIG. 5 or the CT images of FIG. 4 that an augment may be needed to reliably and securely install the implant cup in the position planed in step 302. An option can be selected via the graphical user interface 400 to include an augment. FIGS. 6-7 show views of the graphical user interface 400 that show a virtual augment model 600 and which facilitate selection of a desired placement of the virtual augment model 600. As shown in FIG. 6, the virtual augment 600 is visualized in a position relative to the virtual bone model 502 and the virtual implant cup 404 in a 3-D opaque view. As shown in FIG. 7, the virtual augment 600 is visualized in a position relative to the virtual bone model 502 in a translucent view and in two CT image views. FIGS. 6-7 are described in further detail below.

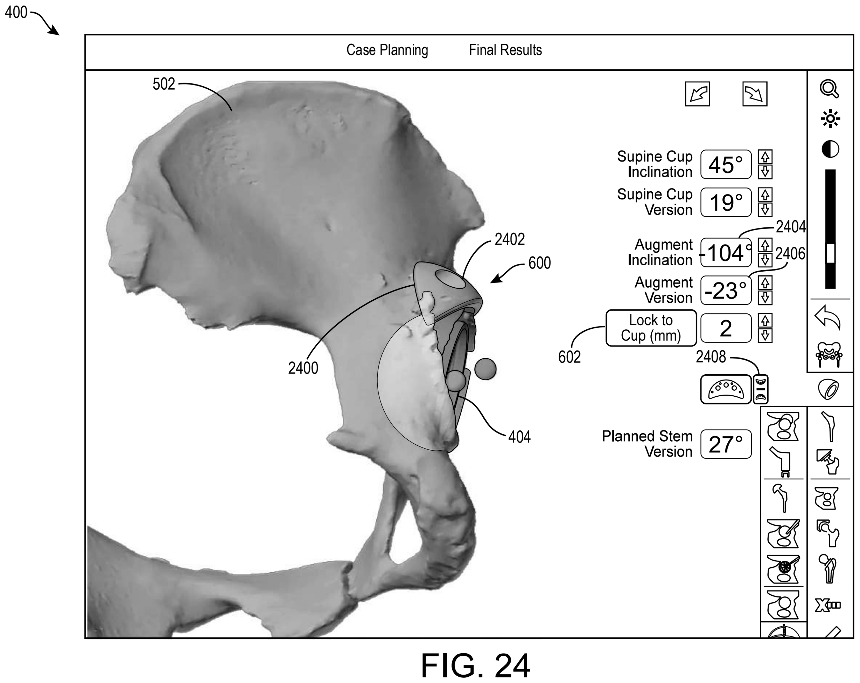

The graphical user interface 400 may include a warning message to indicate that an orientation of the virtual augment 600 violates a rule. For example, the rule may include an acceptable range of orientations of an augment required to support cutting a bone during a hip arthroplasty procedure. Step 304 can include comparing the orientation of the virtual augment 600 to the acceptable range of orientations to determine whether the virtual augment is oriented within the acceptable range of orientations and generating the warning if the virtual augmented is oriented outside the acceptable range of orientations. In some such examples, the graphical user interface 400 may include the warning message to indicate that the virtual augment 600 is improperly oriented (e.g., "upside down," etc.). In some embodiments, the computing system 224 may allow planning the placement of the implant augment to proceed, despite the warning message being displayed on the graphical user interface 400. In some embodiments, steps of process 300 relating to bone preparation are omitted, prevented, abstained from, etc. in scenarios where such a warning is triggered (e.g., to allow planning but not robotically-assisted bone cutting for a "flying buttress" use of an augment). In other embodiments, as described in detail below with reference to FIGS. 24-26, the systems and methods herein are adapted to provide robotic assistance in a workflow dynamically and automatically adjusted based on whether the implant augment is in a standard alignment and use (e.g., with a curved side facing the bone and a flat side facing away from the bone as shown in FIG. 6) or in an inverted (upside-down, flying buttress, flat side facing the bone) alignment.

In most cases, an implant augment has an interior surface that substantially matches an exterior surface of the implant cup, for example having a degree of curvature or radius substantially equal to the exterior surface of the implant cup. The augment is thereby configured to be placed adjacent to the implant cup and to provide structural support for the implant cup.

As shown in FIG. 6, the graphical user interface 400 includes a lock-to-cup button 602. When the lock-to-cup button 602 is selected, the virtual augment 600 is restricted to a pre-defined spacing relative to the virtual cup 404. For example, the virtual augment 600 may be positioned such that the virtual augment 600 is approximately two millimeters from the virtual cup 404. This spacing provides a volume which may be filled with cement or other adhesive during the procedure to couple the augment to the cup. As shown in FIG. 6, the graphical user interface 400 includes an array of control buttons 604 that can be selected to alter the rotation, version, and inclination of the virtual augment 600 while preserving the pre-defined spacing relative to the virtual cup 404. Accordingly, step 304 may include restricting the planned placement of the implant augment to a pre-defined spacing relative to the planned position of the cup.

As shown in FIG. 7, the graphical user interface 400 shows a representation of the virtual augment 600 and the virtual bone model 502 without the virtual cup 404. As shown in FIG. 7, the graphical user interface 400 may facilitate a surgeon in evaluating the contribution of the virtual augment 600 to formation of a surface for receiving the cup. CT views 704 show two-dimensional views of the virtual augment 600 relative to CT images collected of the patient's hip. The CT images may show bone density, a previously-implanted cup, other implant components (e.g., screws, plates, etc. used to treat traumatic injury), and/or other useful information. The graphical user interface 400 of FIGS. 6-7 thereby facilitate planning of the implant augment relative to the implant cup and the pelvis. The graphical user interface 400 may also facilitate planning of screw trajectories of the implant and the augment, so that such screw trajectories are considered/planned simultaneously. This may ensure that the augment and implant cup are positioned such that the screws will not interfere with one another or with any existing hardware (e.g., trauma screws/plates). The screw trajectories may also be visualized relative to bone density to ensure adequate screw fixation is achieved.

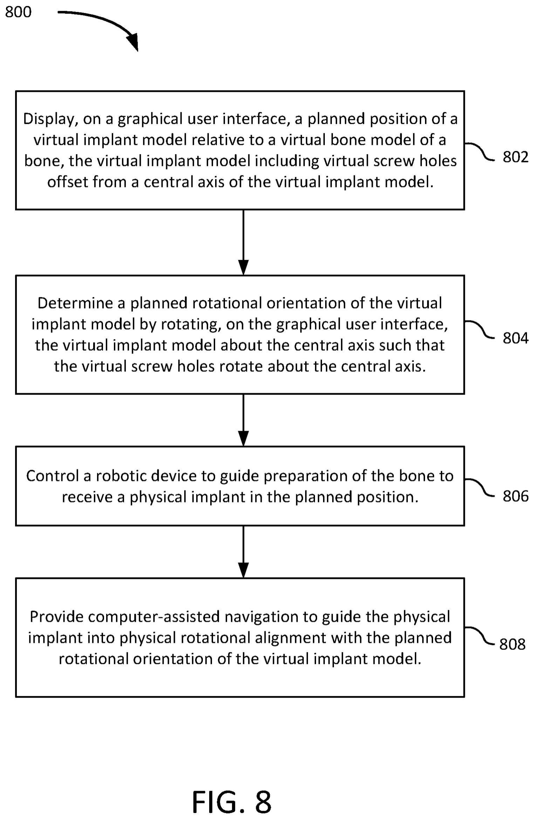

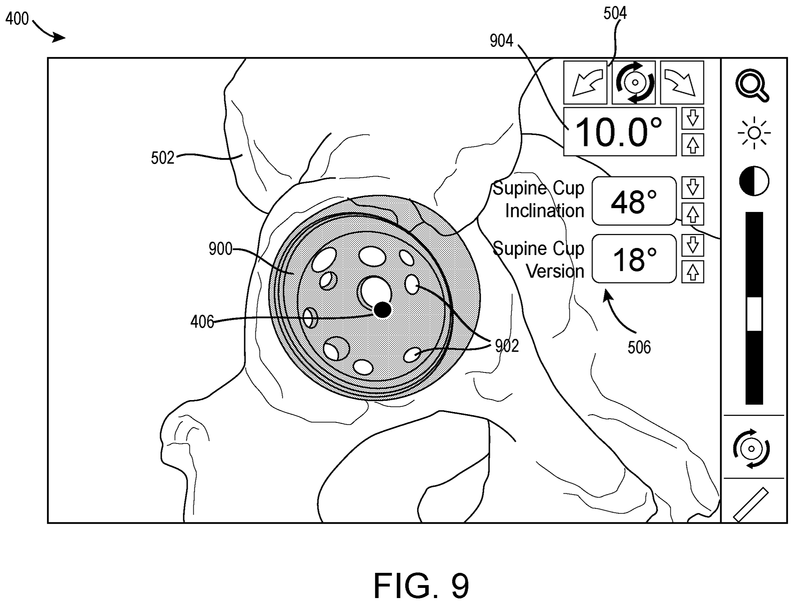

As shown in FIGS. 8-11, the graphical user interface 400 may also facilitate planning the position of the implant cup by planning a rotational orientation of the implant cup, such that the planned rotational orientation enables optimal screw hole planning. Planning the rotational orientation of the implant cup may include "clocking" the implant cup by rotating the implant cup about a central axis of the implant cup. As illustrated in FIG. 8, process 800 for facilitating implant clocking and screw planning during process 300 may begin by displaying a planned position of a virtual implant model relative to a virtual bone model of a bone at step 802. For example, as shown in FIG. 9, a virtual implant model 900 may be displayed on the graphical user interface 400 relative to the virtual bone model 502. The virtual implant model may otherwise include the virtual cup 404 and, in such instances, the planned position of the virtual implant model may include the pose of the cup relative to the pelvis planned at step 302 of process 300.

FIG. 9 shows a view of the graphical user interface 400 that shows a virtual implant model 900 with a plurality of virtual screw holes 902 and which facilitates planning the rotational orientation of the virtual implant model 900. In some embodiments, the plurality of virtual screw holes 902 represent a plurality of openings within an implant (i.e., the cup 28a). Each of the plurality of openings in the implant is configured to receive a screw for securing the implant to the bone. In some instances, as shown in FIG. 9, each of the plurality of virtual screw holes 902 may be offset from the center point 406 of the virtual implant model 900.

As shown in FIG. 9, the virtual implant model 900 is visualized in a position relative to the virtual bone model 502 in a 3-D view. The graphical user interface 400 is shown as including control arrows 504 that can be selected to alter the rotation, version, and inclination of the virtual implant model 900 in order to obtain the desired rotational orientation of the virtual implant model 900. As shown in FIG. 9, the graphical user interface 400 may include a degree box 904 to display how many degrees by which the virtual implant model 900 rotates when a user engages with the control arrows 504. For example, as shown, each click of the control arrows 504 rotates the virtual implant model 900 by 10.0° to the right and/or to the left. In some embodiments, a user may adjust (i.e., increase and/or decrease) the number of degrees displayed in the degree box 904 to allow for finer adjustments and/or larger/faster rotations of the virtual implant model 900. As shown in FIG. 9, the graphical user interface 400 includes arrows (i.e., an up arrow and a down arrow) next to the degree box 904 that allow the user to increase (i.e., using the up arrow) and decrease (i.e., using the down arrow) the number of degrees by which the virtual implant model 900 is "clocked" with each click of the control arrows 504. The graphical user interface 400 also includes the data fields 506 that show various information that may be of interest to the user, for example, pelvic tilt, cup inclination, cup version, stem version, combined version, and superior, medial, and anterior distances.

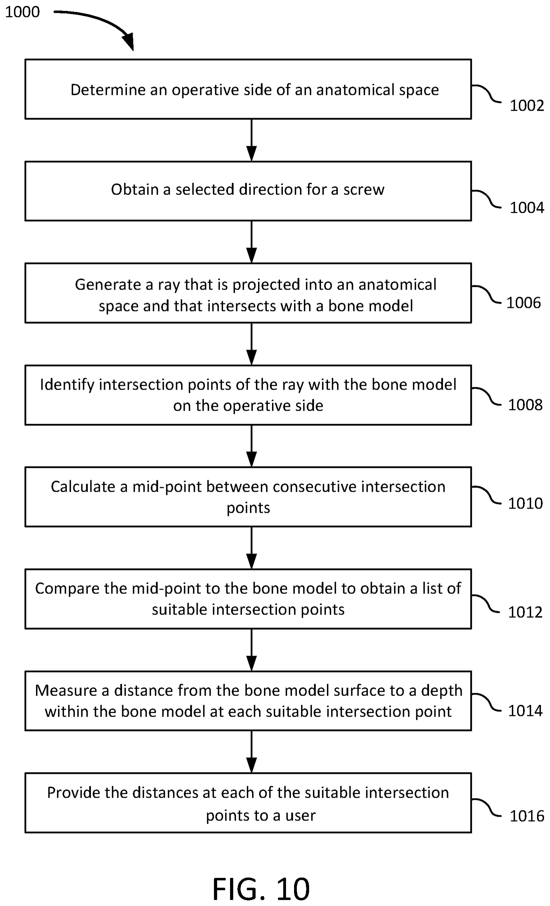

At step 804, a planned rotational orientation of the virtual implant model is determined by rotating, on the graphical user interface, the virtual model about the central axis such that the virtual screw holes rotate about the central axis. For example, a user of the surgical system 200 (i.e., a surgeon performing the hip arthroplasty procedure, as described herein), may use the control arrows 504 to rotate the virtual implant model 900 about the center point 406 on the graphical user interface 400. As shown in FIG. 9, each click of the control arrows 504 may rotate the virtual implant model 900 about the center point 406 by 10.0°. In this way, as the user rotates the virtual implant model 900 about the center point 406, the plurality of virtual screw holes 902 may similarly rotate about the center point 406 to obtain poses of the plurality of virtual screw holes 902 relative to the virtual bone model 502, as described below with reference to FIGS. 10-11.

After the planned rotational orientation of the virtual implant model is determined at step 804, process 800 may continue by controlling a robotic device to guide preparation of the bone to receive a physical implant in the planned position at step 806. In some embodiments, step 806 may be performed in a similar or identical manner as steps 308 and 310 of process 300, as described below. For example, at step 806, the robotic device 220 may be controlled to ream the acetabulum to prepare a surface of the pelvis to receive the physical implant according to the planned position. In some embodiments, the physical implant may be the cup 28a.

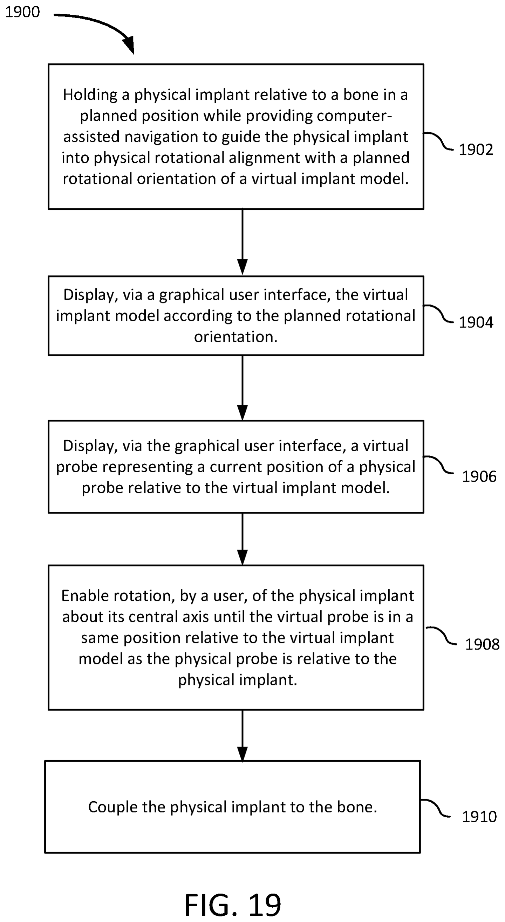

Step 808 includes providing computer-assisted navigation to guide the physical implant into physical rotational alignment with the planned rotational orientation of the virtual implant model. The physical rotational alignment refers to a rotational alignment of the physical implant (i.e., the cup 28a) in real space, rather than the rotational orientation depicted on the graphical user interface 400 by the virtual implant model 900. In some embodiments, the computer-assisted navigation may include one or more techniques as described herein with reference to the robotic device 220, the tracking system 222, and/or the computing system 224. For example, various navigation features for guiding the physical implant into physical rotational alignment with the planned rotational orientation of the virtual implant model are described below with reference to FIGS. 19-21.

As mentioned above, as the user rotates the virtual implant model 900 about the center point 406, the plurality of virtual screw holes 902 may similarly rotate about the center point 406 to achieve an optimal position of each of the plurality of virtual screw holes 902 relative to the virtual bone model 502. FIG. 10 illustrates a process 1000 for estimating a maximum screw length/trajectory for a screw receivable by a screw hole within an implant such that the screw does not protrude from a patient's bone. In the scenario of a hip arthroplasty procedure, various intricacies of the hip joint, including uncertain bone dimensions, complicate screw insertion during the procedure. Moreover, this lack of certain information makes accurately predicting an ideal screw length difficult. Using a screw that protrudes from a patient's bone may cause complications such as damage to a patient's tissues, nerves, and arteries, which can ultimately hinder leg mobility. The harm caused to these sensitive structures in the patient's anatomy results in pain, reduced function, limited mobility, and other serious medical complications. Advantageously, a hip arthroplasty procedure, as described herein, may include determining an optimal screw length as outlined by process 1000.

As shown in FIG. 10, the process 1000 begins by determining an operative side of the anatomical space at step 1002. The operative side of the anatomical space is defined as the space on a side of the bone model facing the planned implant. In some embodiments, the operative side of the anatomical space may be determined based on various CT scan data. For example, the operative side may be determined based on the CT views 704 of the graphical user interface 400. The various CT scan data includes specific landmarks within the anatomical space and may be used to establish a mid-plan to differentiate one side of the anatomical space from the other side of the anatomical space. The specific landmarks may be used to determine the operative side from the sides of the anatomical space differentiated by the mid-plane.

After determining the operative side of an anatomical space, process 1000 continues with obtaining a selected direction for a screw at step 1004. The screw may refer to a screw receivable by a first screw hole of the plurality of virtual screw holes 902. In some embodiments, the direction may be obtained based on the poses of the plurality of virtual screw holes 902 as defined by the planned rotational orientation of the virtual implant model 900 during step 804 of process 800 (e.g., a direction normal to the surface of the virtual implant model 900 at a selected screw hole of the plurality of virtual screw holes 902, a direction defined in the virtual implant model 900 as an axis of a selected screw hole, etc.). The direction may also be obtained based on a probe position (i.e., a probe tracked by the tracking system 222) and/or a user-selected position received via the graphical user interface 400. For example, a user may click on one of the virtual screw holes 902 as shown on graphical user interface 400, and the computing system 224 may be configured to proceed with calculating the maximum screw length for that virtual screw hole based on a current rotational orientation of the virtual implant model 900 shown on the graphical user interface 400 and data stored in the virtual implant model 900 relating to an axis or direction associated with the virtual screw hole. Similarly, the user may be configured to use the probe to pick (e.g., place the probe within a screw hole while clicking a button, pushing a foot pedal, etc.) on a screw hole within a physical implant that corresponds to a virtual screw hole among the plurality of virtual screw holes 902 on the virtual implant model 900.

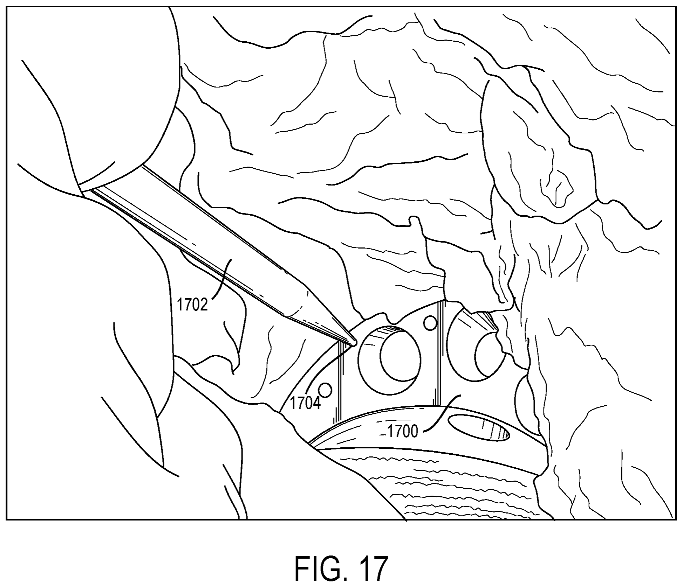

For example, the selected direction may be projected from a tip of a virtual probe and along its negative axis (i.e., away from the tip of the virtual probe) on the graphical user interface 400 that intersects with the virtual bone model 502. In some embodiments, such a projection can correspond to virtualization of projecting a ray from the tip of a physical probe and along the negative axis of the physical probe as may be positioned in real space, such that the user can select a direction by orienting the physical probe. The probe is an instrument used to navigate and interact with anatomical structures of interest, both in real space and virtual models. For example, in some embodiments, the probe is the navigation probe 1702, as described in greater detail below with reference to FIG. 17.

After obtaining the selected direction for the screw, process 1000 further includes at step 1006, generating a ray along the selected direction that is projected into an anatomical space and that intersects with a bone model. The ray extends into (through) the bone model along the selected direction. In some embodiments, the ray is a straight line.

As illustrated in FIG. 10, the process 1000 includes identifying intersection points of the ray with the bone model on the operative side at step 1008. For example, the intersection points may be virtual indications on the graphical user interface 400 where a ray generated by a virtual probe tip intersects with the virtual bone model 502. In this example, these intersection points represent where the ray meets bone surface represented by the virtual bone model 502. Intersection points on a non-operative side (i.e., a side of the bone model that is not receiving the planned implant) may be disregarded, such that the intersection points can be considered as points where the ray enters the bone model (including at points across an open space or gap within the bone model).

Step 1010 of the process 1000 includes calculating a mid-point between consecutive intersection points from the intersection points identified at step 1008. Based on the mid-points, process 1000 continues with comparing the mid-points to the bone model to obtain a list of suitable intersection points at step 1012. In some embodiments, comparing the mid-points to the bone model may include assessing whether a mid-point lies within the bone model or whether a mid-point lies outside of the bone model. If the mid-point lies within the bone model, the screw may be cast/projected to a second intersection point (i.e., the further intersection point from the implant) of the consecutive intersection points used to calculate the mid-point. If the mid-point lies outside of the bone model, however the screw may be cast/projected to a first intersection point (i.e., a nearer intersection point to the implant) of the consecutive intersection points used to calculate the mid-point. Each of the first intersection points and/or second intersection points may be compiled into a list of suitable intersection points.

At step 1014, a distance may be measured from the bone model surface to a depth within the bone model at each suitable intersection point (i.e., each of the first intersection points and/or second intersection points identified at step 1012, as described above) from the list of suitable intersection points obtained at step 1012. The distance may be measure to a depth within the bone model such that a screw may not protrude from the bone model. In some embodiments, the distance also includes an offset based on a depth of the screw hole (e.g., such that the distance represents a length of a screw to be inserted including a portion that will be seated within the screw hole). The distances corresponding to each suitable intersection point may be provided to a user (i.e., a surgeon performing a hip arthroplasty procedure) at step 1016. For example, the distances may be provided to the user via the graphical user interface 400. In some embodiments, a maximum distance for the screw may be provided to the surgeon as a guideline for choosing an appropriate screw length (i.e., physical obtaining a physical screw from a set of screws of different lengths) during surgery (e.g., a hip arthroplasty procedure, as described herein).

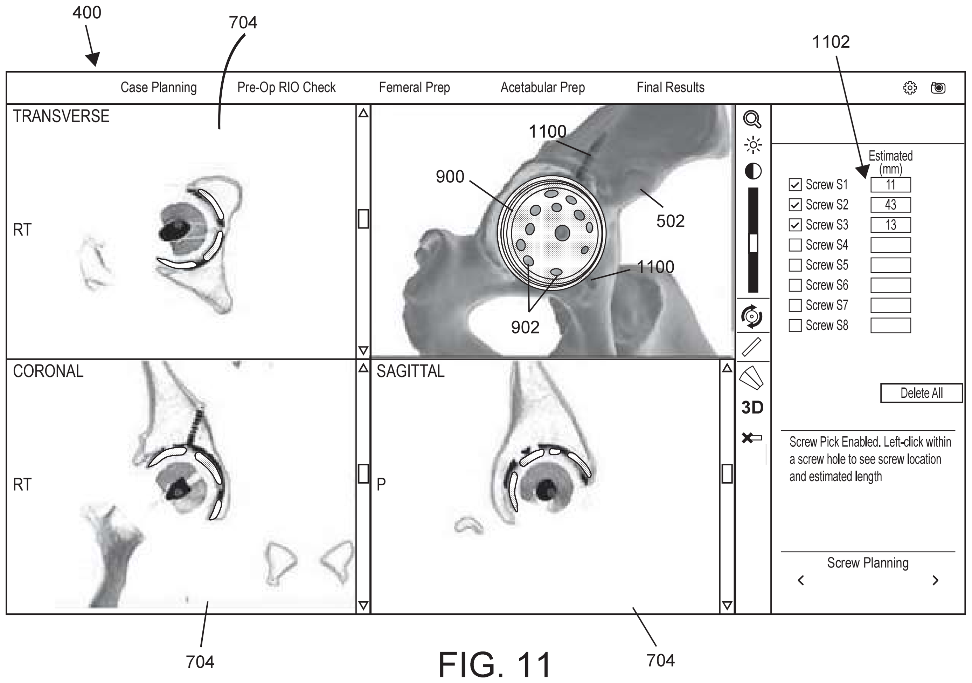

For example, as shown in FIG. 11, the graphical user interface 400 may be used to display the maximum screw lengths for each of the plurality of virtual screw holes 902 according to a rotational orientation of the virtual implant model 900. The maximum screw lengths may be calculated using the process 1000, as described above. The graphical user interface 400 may include the CT views 704 and an illustration of the virtual implant model 900 relative to the virtual bone model 502. As shown in FIG. 11, the virtual implant model 900 may include the plurality of virtual screw holes 902 and one or more virtual screws 1100 receivable by one or more of the plurality of virtual screw holes 902. The maximum screw lengths may be displayed as screw data 1102 for a screw receivable by each of the plurality of virtual screw holes 902. In some embodiments, the screw data 1102 may include a plurality of selectable elements representing a plurality of screws receivable by the plurality of virtual screw holes 902 such that a user (i.e., a surgeon performing a hip arthroplasty procedure) may click on a selectable element included in the screw data 1102 to receive the maximum screw length for a screw receivable by a particular screw hole. Furthermore, as a user rotates the virtual implant model 900 via the graphical user interface 400, the screw data 1102 may update according to an updated rotational orientation of the virtual implant model 900 such that the maximum screw lengths are calculated based on a current orientation of the virtual implant model 900 as displayed on the graphical user interface 400.

By determining the maximum screw lengths as described by process 1000 and using the graphical user interface 400, the rotational orientation of the virtual implant model 900 during step 804 of process 800 may be determined such that the plurality of virtual screw holes 902 fall in an acceptable position relative the virtual bone model 502. The acceptable position allows for a screw with a determined maximum screw length to be received by one of the virtual screw holes 902 without protruding from the virtual bone model 502. Similarly, after the rotational orientation of the virtual implant model 900 is determined at step 804, determining the maximum screw lengths as described by process 1000 and using the graphical user interface 400 ensures that a user of the surgical system 200 (i.e., the surgeon performing the hip arthroplasty procedure) is aware of the screw length to use such that the screw does not protrude from the bone.

Steps 302 and 304 can thereby result in a planned pose of the implant augment and a planned pose of the implant cup. Such planning (i.e., steps 301-304) may occur pre-operatively and/or intraoperatively. The remaining steps of process 300 occur intraoperatively, i.e., during the surgical procedure.

At step 306, a registration process is executed to register the relative positions of the patient's pelvis, the surgical tool(s), the robotic device, and/or other tracked probes or instruments. For example, a probe may be tracked by the tracking system 222 and touched to various points on the pelvis to determine a pose of the pelvis. Various registration methods are described above with reference to FIG. 2.

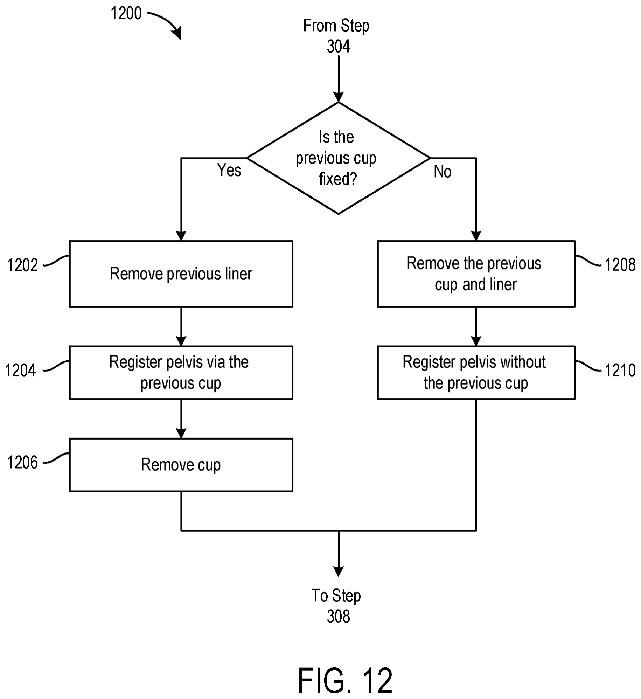

In the case of revision hip arthroplasty procedures, different registration workflows may be used depending on whether the previously-implanted cup is loose or fixed. FIG. 12 shows a flowchart of a process 1200 for registration in revision hip arthroplasty procedures, according to an exemplary embodiment.

As illustrated in FIG. 12, if the previous cup is fixed, the liner of the previous implant (i.e., implanted in a previous procedure) is removed at step 1202. At step 1204, the location of the pelvis is registered via the previous implant cup, which is fixed to the pelvis. For example, a tracked probe can be touched to various locations on the previous implant cup to determine a pose of a surface of the previous implant cup. As another example, intra-operative imaging (e.g., x-ray) may be used to determine a pose of a surface of the previous implant cup. Because the geometric relationship between the previous implant cup and the pelvis is fixed and known from the medical images received at step 301, such data can be used for registration of the pelvis. A tracked probe and/or intraoperative imaging may also be used to locate and register existing hardware (e.g., trauma screws/plates) to facilitate avoidance of such structures during a procedure (e.g., by creating virtual control objects around the located positions of such structures). Following registration, the previous cup is removed at step 1206 to allow the revision implant to be installed. In some embodiments, haptic guidance is used to facilitate removal of the previous (primary, existing) implant, for example as described in U.S. Patent Application 20180014891. For example, a virtual control object can be generated by referencing a library of implant designs to determine a geometry of the relevant implant, identifying the edges of the previous implant using a probe, and generating haptic boundaries based on the probed edges.

Also as illustrated in FIG. 12, if the previous cup is loose (i.e., not fixed), the previous cup and liner are removed at step 1208 prior to registration of the pelvis. At step 1210, the pelvis is registered without the previous cup. For example, a probe may be touched to various points around or in the region from which the previous cup was removed.

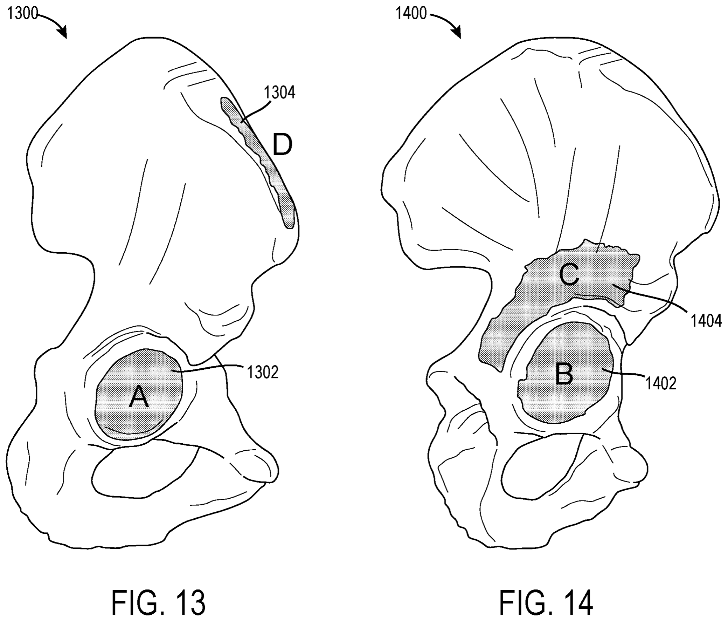

To further illustrate the registration of step 306 according to some embodiments, FIGS. 13-14 depict regions of the pelvis that may be used for registration in various scenarios. FIG. 13 illustrates a virtual bone model 1300 that includes a fixed cup, while FIG. 14 illustrates a virtual bone model 1400 in which a loose cup has been removed, leaving an approximated, smooth surface. FIGS. 13-14 include demarcation of several registration regions, shown as region A 1302, region B 1402, region C 1404, and region D 1304.

In a scenario with a fixed cup, registration points (i.e., points touched by a tracked probe and used for registration) can be taken in region A 1302, which corresponds to a surface of the previously-implanted fixed cup. Such points may be particularly reliable and accessible, as region A 1302 is exposed during surgery to allow for removal of the previously-implanted cup. Other points may also be taken, for example in region D 1304 (along the iliac crest) and/or region C 1404 (above the acetabulum).

In a scenario with a loose cup, registration points can be taken in region B 1402, which corresponds to an acetabular surface exposed when the loose cup is removed from the patient. For example, registration points may be taken around a rim of region B 1402. The reliability of such points may be dependent on the accuracy of the segmentation of step 301 in differentiating the surface of the bone in the pre-operative imagery from the loose cup, which is removed to expose the surface of region B 1402. In some embodiments, registration of the pelvis is achieved in the loose cup scenario without using acetabular registration points (without using registration points in region A 1302 or region B 1402) and by using extra-acetabular registration points (e.g., points in region C 1404 and/or region D 1304).

Registration as conducted at step 306 thereby facilitates a mapping of the actual pose of the pelvis in real space to a virtual position of the virtual bone model 502 in virtual space. The virtually-planned poses of the virtual implant augment and the virtual implant cup can then also be associated with real poses in real space (e.g., relative to a coordinate system used by the tracking system 222).

The primary cup (i.e., the existing implant) can then be removed using standard techniques. In some cases, removal of the primary cup may result in an unexpected defect cavity which was not accounted for in the original surgical plan. In such cases, the tracked probe may be used to define a contour (size, shape, pose, etc.) of the defect cavity, for example by tracking the location of the probe as the probe is touched to various positions on the surface of the defect cavity, traced/painted along the defect cavity, etc. The virtual bone model may then be updated to include a virtual representation of the defect cavity, so that the virtual bone model substantially matches the actual form of the bone after primary cup removal. The surgical plan can then be adjusted to account for the defect cavity, for example by modifying a size or pose of an augment. Intra-operative registration and bone model updates can also be used to correct for voids from a segmentation process or clarify regions of scatter in the original imaging (e.g., CT images).

Similar updates may be made in response to identification of other features that may be located and registered intra-operatively, for example poor bone stock, cysts, etc. In some embodiments, custom virtual control boundaries are automatically generated intra-operatively based on the tracked positions of a probe moved by a user to positions indicating the location of a feature desired to be resected (e.g., a cyst). The robotic device 220 can then be controlled based on the custom virtual control boundary to resect the identified feature.

Additionally, in some embodiments, the virtual bone model may be updated following an initial resection (e.g., osteophyte resection). For example, a cutting accessory (e.g., attached to the robotic device 220) may be tracked relative to the bone as the cutting accessory is used to remove an osteophyte or other feature. Based on the tracked movement of the cutting accessory, the virtual bone model can be automatically updated to include the modifications made by the cutting accessory by removing the portions of the virtual bone model corresponding to the resected features. The virtual bone model can thereby be updated to accurately represent the post-resection bone surface without reimaging. The surgical plan for remaining steps of the procedure can be updated based on the updated virtual bone model, or other interventions can be planed (e.g., bone graft to fill a void, etc.).

At step 308, the robotic device 220 is controlled to ream the acetabulum to prepare a surface of the pelvis to receive the cup in the planned pose. For example, a virtual control object may be generated based on the planned pose of the cup (referred to herein as the "cup virtual control object"). For example, the cup virtual control object may include a surface corresponding to an exterior surface of the cup and arranged in the planned pose of the cup. Such a surface of the cup virtual control object defines a planned bone modification, e.g., a resulting configuration of the bone after a machining (e.g., reaming) process such that the bone is prepared to receive the cup implant in the planned pose.

The robotic device 220 may be controlled at step 308 using the cup virtual control object. In some embodiments, the robotic device 220 executes autonomous movements as guided by the cup virtual control object to move and operate the surgical tool 234 to ream the pelvis to prepare the pelvis to receive the cup in the planned position. In other embodiments, the robotic device 220 provides haptic feedback to a user to constrain the surgical tool 234 within the cup virtual control object as a user manipulates the surgical tool 234 to ream the pelvis to prepare the pelvis to receive the cup in the planned position. These and other possible control modalities are described in detail above with reference to FIG. 2.

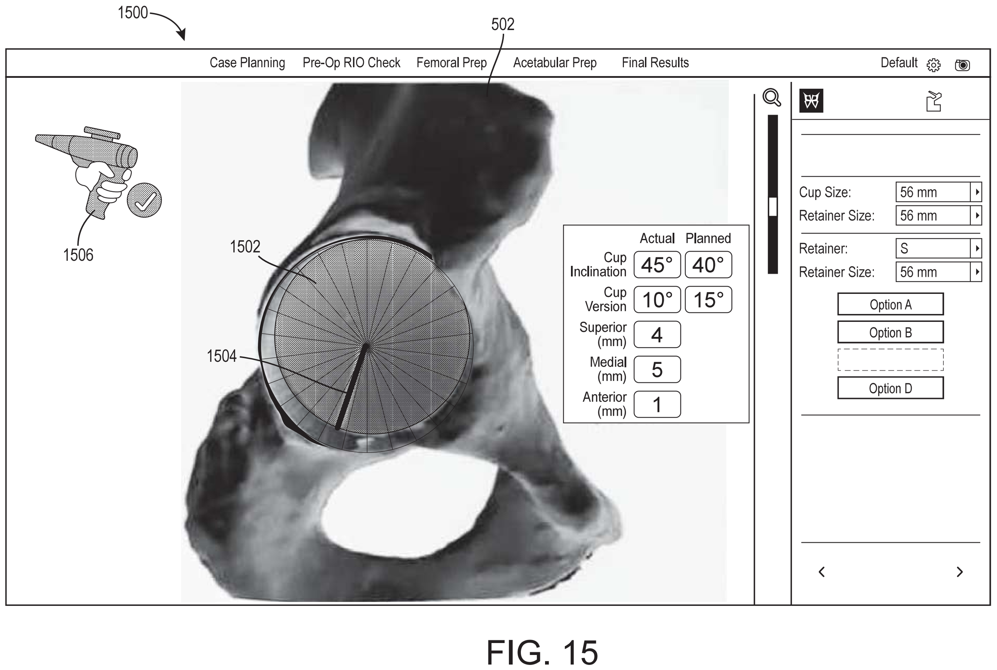

FIG. 15 shows an example of a graphical user interface 1500 that may be generated by the processing circuit 260 and displayed on the display 264 to facilitate execution of step 308, for example an in embodiment where the robotic device 220 is a haptic device. The graphical user interface 1500 shows the virtual bone model 502 with a color-coded (e.g., green) or shaded region 1502 indicating areas of the bone that are to be removed in accordance with the surgical plan. An arrow 1504 indicates a current orientation and center point of the surgical tool 234. A tool indicator 1506 indicates that the surgical tool 234 is currently operating (e.g., that the reamer is rotating).

The processing circuit 260 is configured to update the graphical user interface 1500 in real time using the tracked poses of the pelvis and the surgical tool 234 from the tracking system 222. For example, the color-coded or shaded region 1502 may be reduced in size as the tracking data indicates that the cutting accessory of the surgical tool 234 (e.g., the head of a reamer tool) passes through the corresponding area of the bone. Completion of the planned bone modification corresponds to full consumption (reduction to nothing, erasure, etc.) of the color-coded or shaded region 1502.

The virtual control object may also be indicated on the graphical user interface 1500. In some cases, the processing circuit 260 may provide a different color-coding (e.g., red) to indicate areas where data from the tracking system 222 indicates that surgical tool 234 violated the constraints of the virtual control object and modified the bone beyond the surgical plan.

At step 310, the robotic device 220 is controlled to ream the acetabulum to prepare a surface of the pelvis to receive the implant augment in the planned pose of the implant augment. For example, a virtual control object may be generated based on the planned pose of the augment (referred to herein as the "augment virtual control object"). For example, the augment virtual control object may include a surface corresponding to an exterior surface of the augment and arranged in the planned pose of the augment. Such a surface of the augment virtual control object defines a planned bone modification, e.g., a resulting configuration of the bone after a machining process such that the bone is prepared to receive the augment implant in the planned pose.

In some embodiments, the cup virtual control object and the augment virtual control object are separate virtual control objects and are applied sequentially to execute the surgical plan by first preparing the bone to receive the cup and then preparing the bone to receive the augment. In some cases, the sequence may be reversed, such that the robotic device 220 is controlled to first prepare the bone to receive the augment using the augment virtual control object and then the cup virtual control object is applied to control the robotic device 220 to prepare the bone to receive the cup.

In some such embodiments, a different approach orientation for the surgical tool may be required by the cup virtual control object and the augment virtual control object. The processing circuit 260 may determine completion of the first bone modification (i.e., an end of step 308) and guide the surgical tool from the orientation required by the cup virtual control object into the orientation required by the augment virtual control object, for example using a collapsing haptic boundary, before initiating the second bone modification (i.e., execution of step 310). Additionally, in some embodiments, a change to the surgical tool 234 may be made between steps 308 and 310, for example such that a first reamer head with a first size is used to prepare the cup region and a second reamer head with a second (e.g., smaller) size is used to prepare the bone to receive the augment. The graphical user interface 1500 may display a prompt to make such a change to the surgical tool 234.

In other embodiments, the cup virtual control object and the augment virtual control object are combined as a single virtual control object that includes surfaces corresponding to both the cup and the augment. In such embodiments, steps 308 and 310 can be executed in a unified (simultaneous) manner.

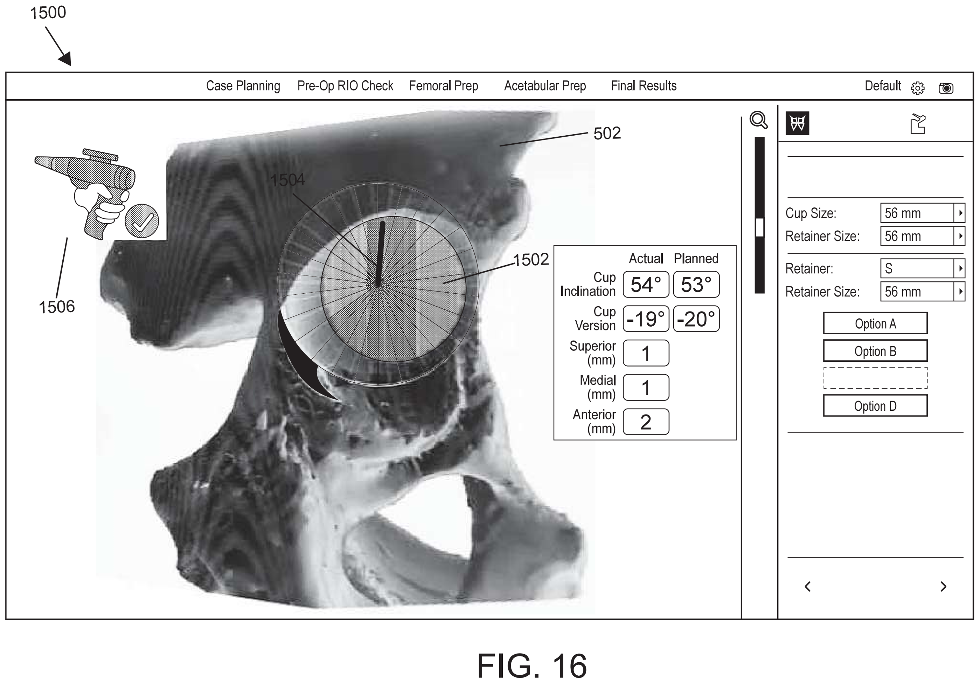

FIG. 16 shows the graphical user interface 1500 as displayed during step 310 in an exemplary embodiment. The graphical user interface 1500 shows the virtual bone model 502 with a color-coded (e.g., green) or shaded region 1502 indicating areas of the bone that are to be removed in accordance with the surgical plan during step 310. The virtual bone model 502 has been modified by the processing circuit 260 to visualize the modifications to the actual bone made during step 308. The arrow 1504 indicates a current orientation and center point of the surgical tool 234. In the example shown, the arrow 1504 has changed orientation relative to the orientation of the arrow 1504 as shown in FIG. 15. The tool indicator 1506 indicates that the surgical tool 234 is currently operating (e.g., that the reamer is rotating).

To facilitate step 308, the processing circuit 260 is configured to update the graphical user interface 1500 in real time using the tracked poses of the pelvis and the surgical tool 234 from the tracking system 222. For example, the color-coded or shaded region 1502 may be reduced in size as the tracking data indicates that the cutting accessory of the surgical tool 234 (e.g., the head of a reamer tool) passes through the corresponding area of the bone. Completion of the planned bone modification corresponds to full consumption (reduction to nothing, erasure, etc.) of the color-coded or shaded region 1502.

Steps 308 and 310 thereby result in a bone (e.g., pelvis) prepared to receive the cup in the pose planned at step 302 and to receive the implant in the pose planned at step 304.

At step 312, the augment is placed in the planned pose and a match between the actual pose of the augment and the planned pose is verified, for example as illustrated in the example embodiment of FIG. 17. As shown in FIG. 17, a surgeon has manually placed the augment 1700 in the surgical site and adjacent the bone in approximately the planned pose. A navigation probe 1702 is shown as touching a point on the augment 1700. The navigation probe 1702 can be tracked by the tracking system 222, such that the tracking system 222 can ascertain a location of a tip 1704 of the navigation probe 1702 relative to other tracked objects, for example the bone modified at steps 308-310. By tracking the navigation probe 1702 as the navigation probe 1702 is touched to multiple points on the augment 1700, a pose of the augment 1700 can be determined by the tracking system 222 and the processing circuit 260. In such embodiments, the processing circuit 260 is configured to compare the tracked pose of the augment 1700 to the planned pose of the augment from step 304. The processing circuit 260 may cause the display 264 to display an indication that the tracked pose of the augment 1700 matches the planned pose of the augment and/or provide guidance for modifying the actual pose of the augment 1700 to bring the tracked pose of the augment 1700 into agreement with the planned pose of the augment 1700. In other embodiments, the augment 1700 may be coupled to a tracked inserter tool, such that the processing circuit can use the tracked pose of the inserter tool to facilitate navigation of the augment to the planned pose. In some embodiments, the inserter tool is supported by the robotic device 220 or another robotic arm such that the inserter tool can hold the augment 1700 in a selected position.

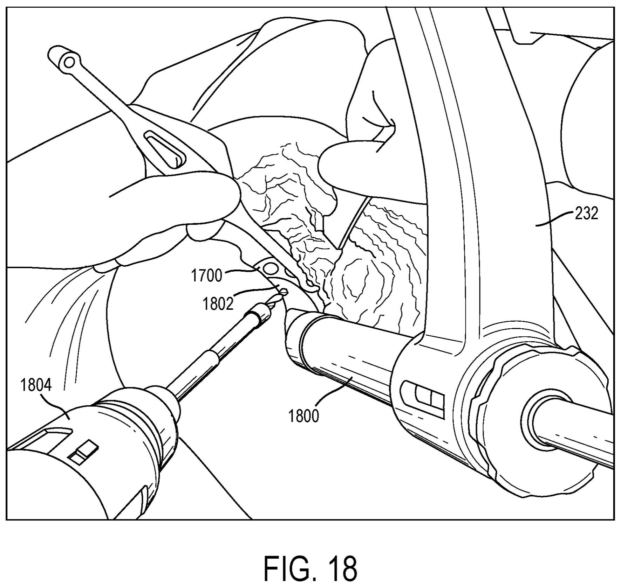

At step 314, the robotic device 220 is controlled to hold the augment in the planned placement while the augment is coupled to the pelvis, for example as illustrated in the example embodiment of FIG. 18. As shown in FIG. 18, the augment 1700 is positioned as described with reference to FIG. 17 and step 312. A holder arm 1800 is coupled to the robotic arm 232 and is shown as holding a trial cup implant 1802. The robotic arm 232 is controlled to force the trial cup implant 1802 against the augment 1700 to push the augment 1700 against the bone, thereby holding the augment 1700 in the planned pose relative to the bone. The augment 1700 can then be coupled to the bone. In the example of FIG. 18, a surgical drill 1804 (e.g., a flexible drill) is used to insert one or more screws through the augment 1700 and into the bone to secure the augment 1700 to the bone in the planned position. The trial cup implant 1802, as held in position by the robotic device 220, can substantially prevent movement of the augment 1700 while the screws are inserted, thereby reducing the number of surgeons or surgical assistants needed to conduct the surgery, improving visibility of the surgical field, and improving accuracy of placement of the augment 1700 relative to the surgical plan. Although a trial cup implant is used in this embodiment, a final cup implant may also be used in step 314.

In other embodiments, at step 314, the augment 1700 is coupled to the holder arm such that the holder arm can be moved by the robotic device 220 to adjust the position of the augment 1700. In such an embodiment, the robotic device 220 is controlled to move the augment 1700 to the planned pose, for example autonomously or by providing haptic feedback to a surgeon. In some embodiments, the surgical drill 1804 is robotically-controlled (e.g., coupled to a second robotic arm) and configured to autonomously insert screws through the augment into the bone in accordance with a surgical plan. In some embodiments, a cutting accessory of surgical tool 234 can be used (autonomously or under haptic guidance) to prepare pilot holes for screw insertion. In some such embodiments, a screw insertion accessory can then be mounted to surgical tool 234 to insert (autonomously or under haptic guidance) bone screws into the pilot holes and through the augment.

At step 316, the implant cup is placed in substantially the planned pose for the implant cup (e.g., slightly spaced from the planned pose in anticipation of step 320 described below). In some embodiments, the cup is manually positioned by a surgeon and that position is checked using a navigation probe as described above for the augment with reference to step 312. In other embodiments, the implant cup is mounted on an impaction arm coupled to the robotic device 220. The robotic device 220 is controlled to move the implant cup to substantially the planned pose, for example autonomously or by providing haptic feedback to a user. For example, haptic feedback may be provided by constraining the position of the implant cup within a virtual control object that collapses (gets smaller, converges) as the implant cup is brought closer to the planned pose, i.e., such that the implant cup can be moved closer to the planned pose but not substantially further away from the planned position relative to a current position. The implant cup is thereby positioned and oriented in substantially the planned pose.

Step 316 may further include clocking the implant cup according to the planned rotational orientation of the implant cup determined at step 804 of process 800. As described above, process 800 concludes with providing computer-assisted navigation to guide the physical implant into physical rotational alignment with the rotational orientation of the virtual implant model at step 808. FIG. 19 illustrates a process 1900 for facilitating guide the physical implant into physical rotational alignment with the rotational orientation of the virtual implant model using computer-assisted navigation.

As illustrated in FIG. 19, the process 1900 begins by holding a physical implant relative to a bone in a planned position at step 1902. In some embodiments, step 1902 may be performed using any of the methods/techniques as described during step 314 and/or 316 of process 300. While the physical implant is held in the planned position, computer-assisted navigation is used to guide the physical implant into physical (i.e., actual) rotational alignment with a planned rotational orientation of the virtual implant model. For example, the planned rotational orientation may include the planned rotational orientation of the implant cup determined at step 804 of process 800 and illustrated by the graphical user interface 400, as described above.

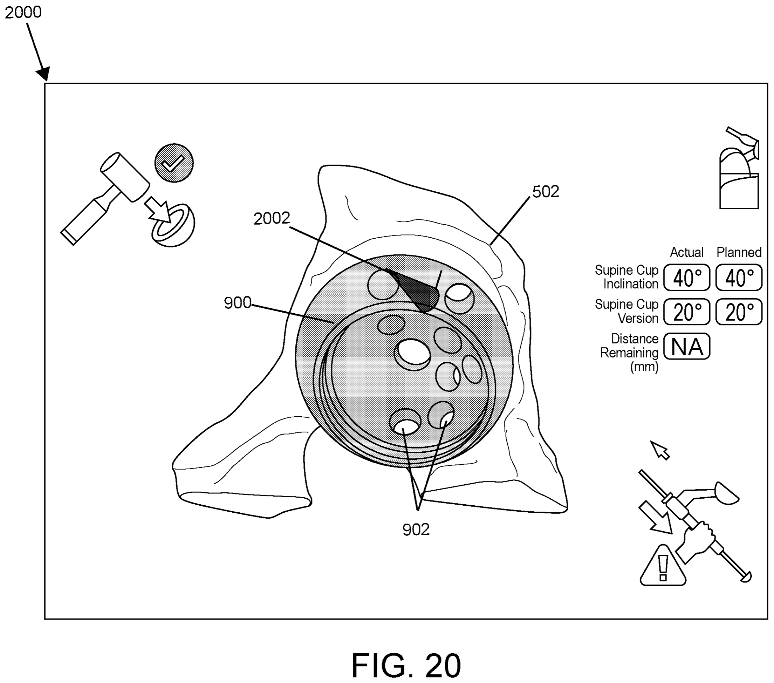

The process 1900 continues by displaying the virtual implant model according to the planned rotational orientation on a graphical user interface at step 1904. The display of the planned rotational orientation may include the display of the planned rotational orientation as illustrated by the graphical user interface 400 in FIG. 9. Step 1906 of process 1900 includes displaying a current position of an optically-tracked probe in the virtual implant model on the graphical user interface. FIG. 20 shows an example of a graphical user interface 2000 that may be generated by the processing circuit 260 and displayed on the display 264 to facilitate execution of process 1900. As shown in FIG. 20, the graphical user interface 2000 includes a display of the virtual implant model 900 relative to the virtual bone model 502 according to the planned rotational orientation. The graphical user interface 2000 also includes a virtual probe 2002. In some embodiments, the virtual probe 2002 is representative of a physical probe being optically-tracked by the tracking system 222. The virtual probe 2002 may be illustrated according to a current position of the physical probe relative to the virtual implant model 900.

As illustrated in FIG. 19, process 1900 continues by enabling rotation, by a user, of the physical implant about its central axis until the virtual probe is in a same position relative to the virtual implant model as the physical probe is relative to the physical implant. That is, the user (i.e., the surgeon) may place the physical probe into a screw hole located on the physical implant. Then, the user may rotate the physical implant, with the physical probe held in the same position relative to the physical implant (i.e., in the screw hole), until the virtual probe is displayed in the same position relative to the virtual implant model as the position of the physical probe relative to the physical implant (i.e., in a virtual screw hole representing the screw hole on the physical implant in which the physical probe is being held).

Upon reaching agreement between the position of the virtual probe relative to the virtual implant model and the position of the physical probe relative to the physical implant model, the user has clocked (i.e., rotated) the physical implant to the planned rotational orientation. For example, FIG. 20 displays the virtual probe 2002 relative to the virtual implant model 900 (i.e., in one of the plurality of virtual screw holes 902). If the position of the virtual probe 2002 relative to the virtual implant model 900 matches the position of a physical probe relative to the physical implant (i.e., in the same screw hole), then the graphical user interface 2000 depicts the virtual implant model 900 according to its planned rotational orientation.

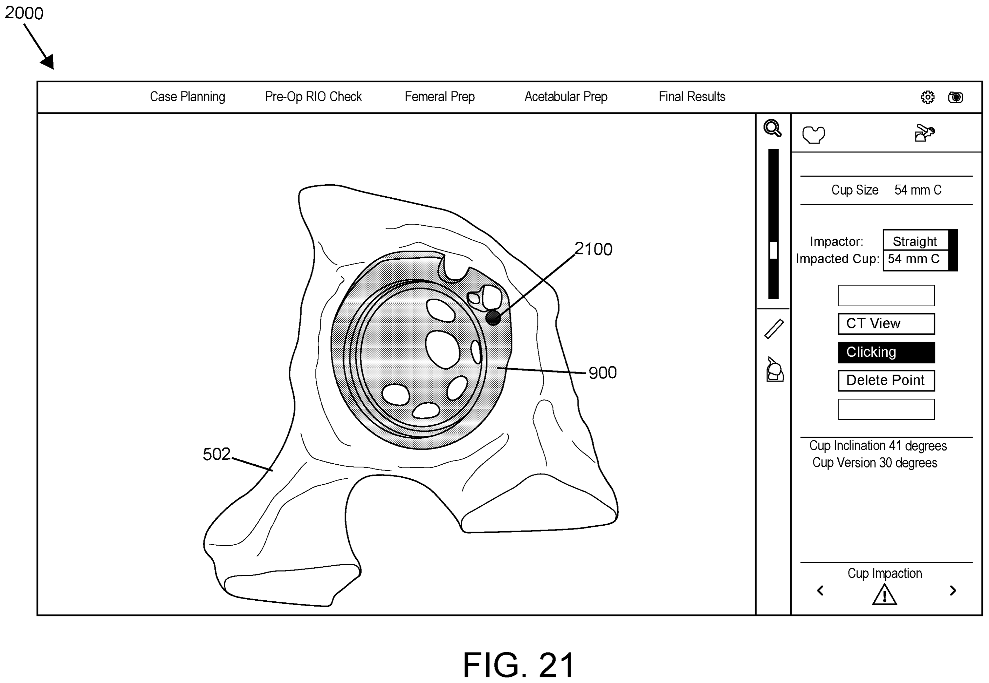

Alternatively or additionally, the user may achieve the planned rotational orientation of the physical implant by selecting (i.e., clicking on) a point on the virtual implant model and aligning a physical probe with the selected point relative to the physical implant. For example, FIG. 21 illustrates the graphical user interface 2000 including a selected point 2100 on the virtual implant model 900. As described with reference to FIG. 20, the user may rotate the physical implant until a position of the physical probe relative to the physical implant aligns with the selected point 2100 relative to the virtual implant model. Upon alignment of the physical probe (as tracked and virtually represented) with the selected point 2100, the system may automatically output an audio chime or other notification to the user that the physical implant has been clocked to the planned orientation.

In some embodiments, the physical implant is manually rotated by a surgeon. In other embodiments, the physical implant is mounted on an impaction arm coupled to the robotic device 220. The robotic device 220 is controlled to rotate the physical implant to substantially the planned rotational orientation. In some embodiments, the achieved rotational orientation may be indicated to the user by a display on the graphical user interface 2000. Alternatively or additionally, the achieved rotational orientation may be indicated to the user by an audio signal. As illustrated in FIG. 19, process 1900 concludes by coupling the implant to the bone, which may be performed after the cup is impacted onto the bone at step 320 and/or after the cement curing at step 322 (which are described below).

At step 318, cement is provided between the cup and the augment. As mentioned above with reference to step 304, the planned pose of the augment is spaced apart from the planned pose of the cup to allow for cement to be included between the cup and the augment to couple the cup to the augment. By following steps 312-316, the actual positions of the cup and the augment also provide space for cement between the cup and the augment. Accordingly, process 300 facilitates use of a predictable, consistent, and preferred (planned, clinically-validated, etc.) amount of cement between the cup and the augment.

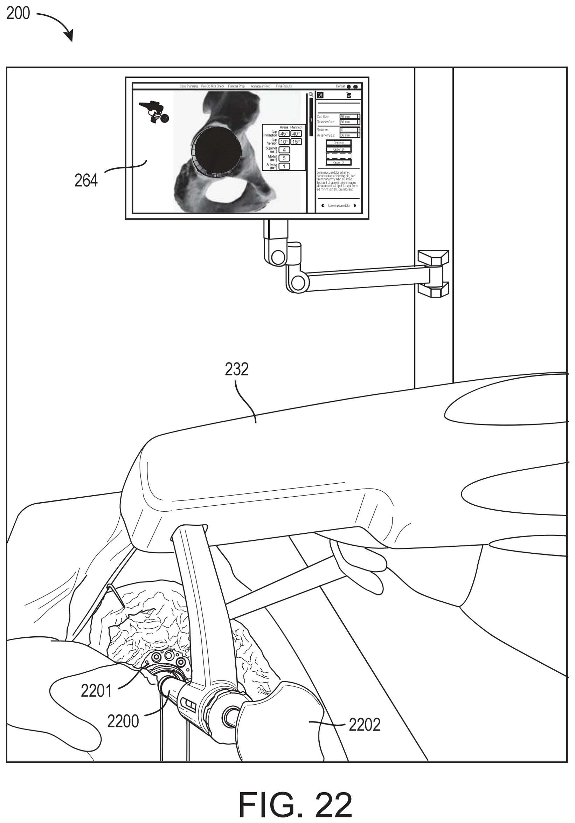

At step 320, the robotic device is controlled to facilitate cup impaction to fix the cup in the planned placement. FIG. 22 shows an example embodiment of the surgical system 200 arranged to execute step 320. As shown in FIG. 22, an impaction device 2200 is mounted on the robotic arm 232. The robotic arm 232 is controlled to align the impaction device 2200 with the planned orientation of the cup and such that a distal end 2201 of the impaction device 2200 is in contact with the cup at substantially the planned position for the cup. FIG. 22 shows the display 264 as providing an indication that the impaction device 2200 is properly positioned for cup impaction. When the surgical system 200 is in the state shown in FIG. 22, the surgeon may provide a blunt force to a proximal end 2202 of the impaction device 2200. The force is transmitted along the impaction device 2200 to impact the cup into the pelvis. This force causes the cup to be driven into the pelvis to substantially fix the cup relative to the pelvis. The robotic arm 232 and information displayed on the display 264 facilitates a surgeon in accomplishing impaction such that the cup is fixed to the pelvis in the planned pose (i.e., as planned at step 302).

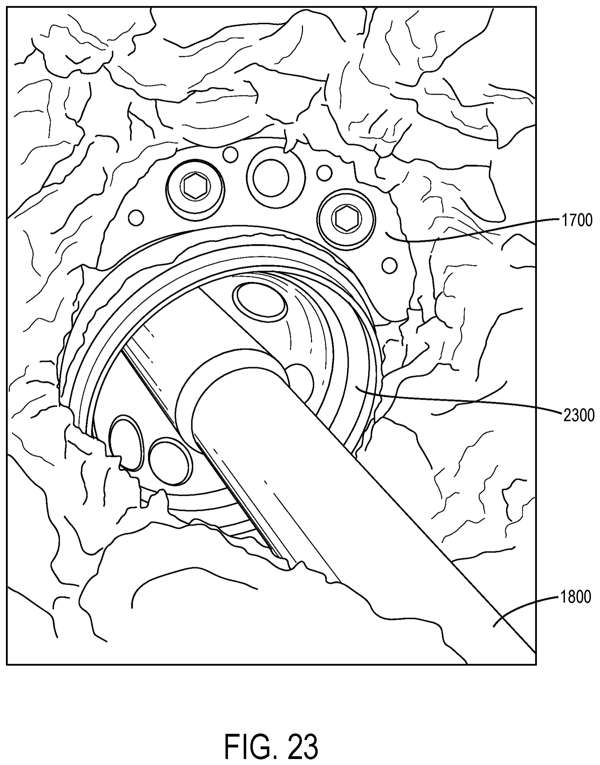

At step 322, the robotic device is controlled to continue to hold the cup in the planned pose for the duration of cement curing (e.g., ten minutes). FIG. 23 illustrates step 322 in an example embodiment, and shows an implant cup 2300 held in position relative to the augment 1700 by the holder arm 1800. The holder arm 1800 may be the same device as the impaction device 2200 or a different device. By automating this holding task, a surgeon or surgical assistant may advantageously become free to accomplish other tasks relating to the surgical procedure. Additionally, robotically-assisted and tracked positioning during cement curing may ensure that the planned geometric relationship between the cup and the augment is achieved. Furthermore, integrity of the cement mantle and unitization of the cup and augment may be optimized because relative movement is minimized as the cement hardens.

Following step 322, the surgical procedure may include coupling the physical implant (i.e., the cup) to the bone (i.e., step 1910 of process 1900). In some embodiments, coupling the implant to the bone includes providing screw length navigation for each of the screws used to couple the implant to the bone. The screw length navigation includes providing the maximum screw lengths determined during process 1000. In some embodiments, the screw length navigation may be provided via the graphical user interface 400, as described above with reference to FIG. 11. In this example, the user may click on any of the plurality of virtual screw holes 902 to receive the maximum screw length for a screw receivable by that screw hole.

In other embodiments, a user may place a physical probe on the physical implant (i.e., in a screw hole). Once the physical probe is in the desired position, the user may provide an input (i.e., a click on the display 264, a push on a foot pedal location on the robotic device 220, etc.) that prompts generation of the maximum screw length of a screw receivable at the position of the physical probe at the time of the input. In this example, the maximum screw length may be in a direction into the bone along the negative axis (i.e., away from the tip) of the physical probe.

In still other embodiments, the maximum screw lengths may be provided using a depth gauge. That is, the user may pre-drill (i.e., prior to insertion of the implant) a hole in the bone for receiving a screw (i.e., drill a pilot hole). The user may insert a depth gauge into the hole to measure its depth and may thereafter use a probe (i.e., tracked by the tracking system 222) to identify two points along the depth gauge. In this example, a line through the two identified points on the depth gauge defines the trajectory of the screw receivable by that pre-drilled hole, and the maximum screw length may be generated according to that defined trajectory. Although described as following step 322, in some embodiments, coupling the implant to the bone and providing the screw length navigation (i.e., step 1910 of process 1900) may follow step 320 (i.e., impact of the cup to the bone), prior to the cement curing at step 322.

In some embodiments, the surgical procedure may proceed following established workflows, for example to position a liner in the cup, to position a femoral implant in the cup, to repair soft tissue proximate the hip joint, and to close the surgical incision. The surgical system 200 may be configured to assist with some or all of these additional steps in various embodiments. Process 300 may thereby improve surgical efficiency and experience for surgeons, reduce the duration of a surgical procedure, and improve patient outcomes by providing accurate placement of augments and cups in accordance with personalized surgical plans.

In some embodiments, data is collected relating to the planning and procedures conducted using the systems and methods described herein. For example, details such as the types of implants used, bone density, ligament balancing measurements, final implant placement (angle, anterior/posterior placement, medial/lateral placement, placement with respect to a joint line, mechanical and anatomic axis positions, etc.), among other possibilities, can be collected during planning of the procedures. Post-operative outcomes may also be collected. The post-operative outcomes may then be compared to the other data to provide insights into improved execution and implementation of the systems and methods described herein.

Referring now to FIG. 24, another example view in the graphical user interface 400 is shown, according to some embodiments. As shown in FIG. 24, the virtual augment 600 is planned in an inverted pose, such that a flat side 2400 is facing the virtual bone model 502 and a curved (convex) side 2402 is facing away from the virtual bone model 502 (see FIGS. 6, 17, and 23 for examples where an augment is oriented in a normal orientation with the flat side 2400 facing away from the bone and the curved side 2402 facing the bone). Accordingly, FIG. 24 shows the virtual augment 600 planned in a "flying buttress" pose, where the virtual augment 600 is able to provide support for portion of the cup 404 protruding from the virtual bone model 502, for example to account for bone loss or degradation or repair of traumatic injury.

FIG. 24 further illustrates that the graphical user interface 400 can include an augment inclination value 2404 and an augment version value 2406. With the augment in the inverted orientation of FIG. 24, the augment inclination value 2404 and augment version value 2406 are shown as negative values. Arrows are provided proximate the values 2404, 2406 which can be clicked (selected, pushed, etc.) by a user to shift the planned pose of the virtual augment 600 in inclination or version. FIG. 24 also shows the lock to cup button 602 selected, such that a gap between the virtual augment 600 and the virtual cup 404 is constrained to a fixed distance (shown as two millimeters) as described above. FIG. 24 also shows the graphical user interface 400 as shown an augment flip button 2408 which, when selected, causes the virtual augment 600 to flip over from the inverted pose shown in FIG. 24 to a normal pose with the curved side 2402 facing the virtual bone model 502.

When the virtual augment 600 is planned with the curved side 2402 facing the virtual bone model 502 (e.g., as in the example of FIG. 6), the patient's physical bone is to be provided during a bone preparation workflow with a curved surface to be mated with the curved side of the physical augment. As described above with reference to steps 308-310, in such embodiments, the reamer used to prepare the bone to receive the cup 404 can also be used to create the curved surface to be mated with the curved side of the implant augment, including in some embodiments in a single reaming stage. However, in the example of FIG. 24 (or other examples where the virtual augment 600 is planned in an inverted pose), the bone is to be provided with a flat surface complementary to (shaped to abut, mate against, etc.) the flat side of the augment. Advantageously, rather than providing error messages or restricting the user from using an augment in an inverted pose, the systems and methods disclosed herein can be adapted to provided robotic assistance for bone preparation in both a normal augment orientation mode and in an inverted augment orientation mode based on automatically detecting which mode is to be used based on the planned pose of the virtual implant augment.

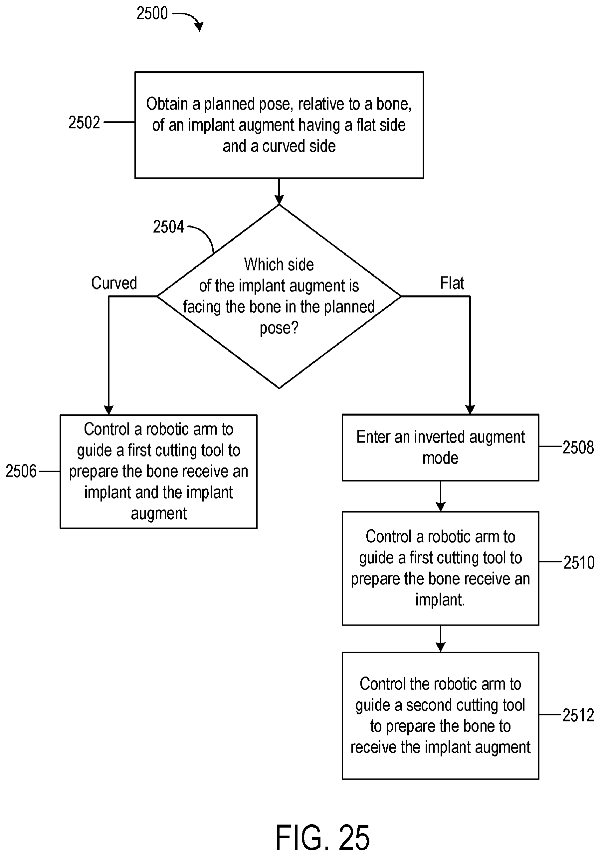

In this regard, referring now to FIG. 25, a flowchart of a process 2500 for preparing a bone to receive an implant augment in an inverted or normal pose is shown, according to some embodiments. The process 2500 can be executed by executed by the surgical system 200 of FIG. 2, for example as part of process 300, in various embodiments.

At step 2502, a planned pose of an implant augment relative to a bone (e.g., a pelvis) is obtained. In the scenarios handled by step 2502, the implant augment has at least one flat side and at least one curved (e.g., convex) side (and, in some examples, an additional side adapted to face an implant being installed). The planned pose of the implant augment can be obtained by displaying, for example via the graphical user interface 400, a virtual augment 600 relative to a virtual bone model 502 and virtual cup 404 and allowing a user (e.g., surgeon) to adjust the pose of the virtual augment 600 relative to the virtual bone model 502 and the virtual cup 404. In some embodiments, step 2502 includes automatically generating the planned pose of the implant augment using an auto-planning algorithm. The planned pose of the implant augment indicates a position and orientation of the implant augment.

At step 2504, a determination is made as to which side of the implant augment is facing the bone in the planned pose. Step 2504 includes determining whether a flat side of the implant augment (e.g., flat side 2400) or a curved side of the implant augment (e.g., curved side 2402) is facing the bone (e.g., virtual bone model 502) and/or determining whether the bone is to be prepared with a flat surface or a curved surface in order to receive the implant augment in the planned pose. Step 2504 can be performed by ascertaining one or more angle of the implant augment in a coordinate system defined by anatomical landmarks on the bone (e.g., a coordinate system determined as described in U.S. Patent No. 11,304,758 granted April 19, 2022) and comparing the one or angles of the implant augment to ranges or thresholds distinguishing angles at which the flat side of the implant augment faces the bone from angles at which the curved side of the implant augment faces the bone. In some embodiments, step 2504 is performed by projecting a ray from the flat side 2400 of the virtual augment 600 (e.g., normal to the flat side 2400) and checking whether the ray intersects the virtual bone model 502; if the ray intersects the virtual bone model 502, the flat side 2400 is determined to be facing the bone, and, if the ray does not intersect the virtual bone model 502, the curved side is determined to be facing the bone. In some embodiments, step 2504 is performed by generating a representation of the bone surface to be created in order to receive the implant augment (e.g., a surface mesh representation, a voxel representation, etc.) and assessing a curvature of the surface to determine whether the bone surface to be created to receive the implant is curved or is substantially flat. Various such techniques for determining which side of the implant augment is facing the bone in the planned pose can be implemented in various embodiments.

If a determination is made that a curved side of the implant augment is facing the bone in the planned pose, process 2500 proceeds to a curved-surface preparation mode in step 2506 where a robotic arm is controlled to guide a first cutting tool to prepare the bone to receive an implant (e.g., acetabular cup) and the implant augment. Step 2506 can include executed steps 306 and 308 described above.

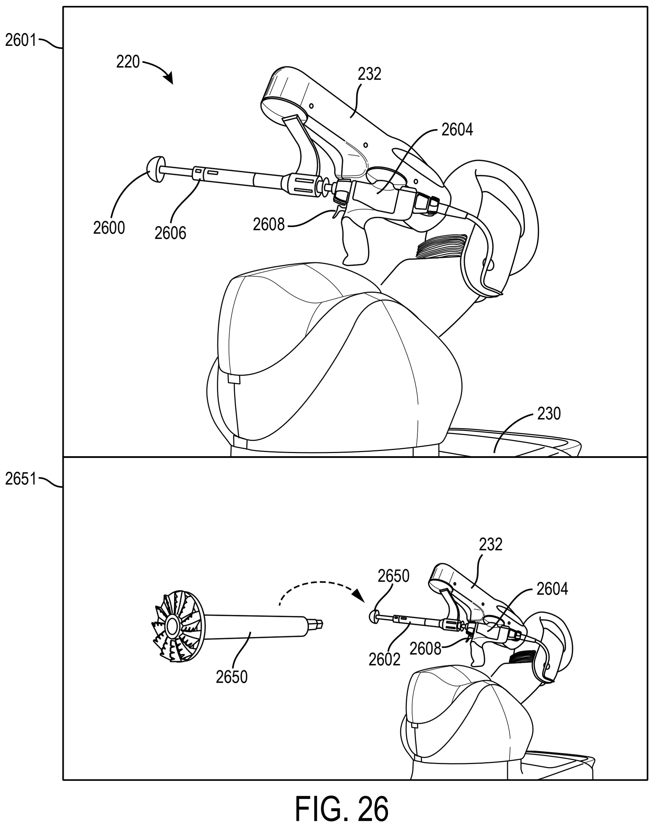

With reference to FIG. 26, as shown in the first frame 2601 of FIG. 26, the first cutting tool used in step 2506 can be a reamer 2600 coupled via shaft 2602 to a power tool 2604 mounted on the robotic arm 232 (which extends from base 230 of the robotic device 220). The reamer 2600, shaft 2602, and power tool 2604 are shown as an example of the surgical tool 234 referred to above. The reamer 2600 has a hemispherical shape and is rotatable by torque provided by power tool 2604, for example in response to depression of trigger 2608 by a surgeon. The reamer 2600 is thereby operable as a first cutting tool adapted to preparing one or more curved bone surfaces to receive an implant (e.g., acetabular cup) and implant augment in step 2506. Accordingly, step 2506 can include operating the robotic device 220 to guide use of the reamer 2600 to prepare the bone to receive the implant and the implant augment (in a normal, non-inverted pose), for example with navigation and force feedback as described above with reference to steps 308 and 310. The implant and implant augment can then be installed on the bone as described above.

If, from step 2504, a determination is made that the flat side of the implant augment is facing the bone, process 2500 process to step 2508 where an inverted augment mode is entered. Step 2508 can include notifying a user, for example via graphical user interface 400, that the implant augment is planned in an inverted (e.g., flying buttress) pose and that the surgical system will provide a bone preparation workflow adapted for providing the bone with a flat surface to receive the implant augment. Such a notification can allow a user to select to go back and adjust the augment position, elect to proceed without robotic assistance, or to process with steps 2510 and 2512 where robotic assistance is provided including for creating a flat surface at the bone to receive the augment in the inverted pose.

In some scenarios (such as shown in FIG. 24), a curved implant (e.g., acetabular cup) is to be installed together with the inverted augment. Accordingly, at step 2510, a robotic arm is controlled to guide the first cutting tool to prepare the bone to receive the implant. The first cutting tool can be the same cutting tool as used in step 2506, for example the reamer 2600. Step 2510 can be executed as described above for step 308.

Because the first cutting tool (e.g., reamer 2600) is adapted to prepare curved surfaces, in the scenarios contemplated herein the first cutting tool is not suitable for modifying the bone to create a flat surface complementary to the flat surface of the implant augment. Accordingly, the inverted augment mode can include instructing a user to change from the first cutting tool to a second cutting tool for use with the robot. With reference to FIG. 26, for example, the reamer 2600 can be replaced with the planer (e.g. Calcar planer) 2650 shown in the second frame 2651 of FIG. 26. That is the reamer 2600 can be detached from the shaft 2602 and the planer 2650 can be attached to the shaft 2602 such that the power tool 2604 is operably coupled to the planer 2650 and can cause rotation of the planer 2650 by exerting torque when a user depresses the trigger 2608. The planer 2650 includes a cutting disk (teeth, cutters, grinders, etc.) arranged to plane a flat surface on a bone such that the flat surface has anormal pointing along the shaft 2602. In some embodiments, the planer 2650 can be selected to have a same radius of curvature as the augment such that creating a planar surface into the bone with the planer 2650 also creates a cavity, depression, etc. complementary to the shape of the implant augment via a single plunge cut by the planer 2650.

Still referring to process 2500 of FIG. 25, at step 2512 the robotic arm is controlled to guide a second cutting tool (e.g., planer 2650, a burr) to prepare the bone to receive the implant augment. That is, the robotic arm is controlled to guide the second cutting tool in creating a planar surface on the bone at the planned pose of the implant augment. Step 2512 can include defining a virtual cylinder (cylindrical haptic object) or line haptic extending normal to the flat bone surface to be created (e.g., normal to the flat side of the virtual augment 600 in the planned pose, aligned with the planed pose of the implant augment). The robotic arm can then operate to constrain the planer 2650 to said haptic object while a user manipulates the planer 2650 to plane the bone and create the flat bone surface. The haptic object may also define a maximum depth of the planer into the bone, thereby causing the robotic device to stop a user from forcing the planer deeper into the bone than necessary to create the planned bone surface that will receive the implant augment in the planned pose. Robotic control and accompanying graphical user interface features for navigation of cutting, updates on status of completion of step 2412 including live-updated models, etc. can be provided as described above for other bone preparation steps.

Advantageously, the approach trajectory of the planer 2650 in step 2512 is substantially similar to the approach trajectory of the reamer 2600 in step 2510, such that the patient position and the gross position of the robotic device 220 can be preserved between steps and registration procedures may not need to be re-performed between such steps, and such that robotic assistance in step 2512 can be provided without substantially increasing an incision size as compared to procedures where no augment is placed.

As shown, the preparation of the flat surface to receive the implant augment in step 2512 is performed after reaming of a curved surface to receive the implant in step 2510. In other embodiments, step 2510 is performed before step 2512. In yet other embodiments, the inverted augment mode entered at step 2508 is provided by using a burr as the cutting tool with the robotic device controlled to use the burr to prepare both the curved surface to receive the implant and the flat surface to receive the augment in a combined burring stage (in such embodiments, step 2506 may still be performed with the reamer 2600) (or in some embodiments, the bur is used as the first cutting tool to prepare the bone to receive the implant in step 2510 and the reamer is used as the second cutting to prepare the bone to receive the augment in step 2512). Once the bone has been prepared to receive both the implant and the implant augment, the implant and implant augment can be installed on the bone, for example as described above with reference to steps 312-322 of process 300.

The construction and arrangement of the systems and methods as shown in the various exemplary embodiments are illustrative only. Although only a few embodiments have been described in detail in this disclosure, many modifications are possible (e.g., variations in sizes, dimensions, structures, shapes and proportions of the various elements, values of parameters, use of materials, colors, orientations, etc.). For example, the position of elements may be reversed or otherwise varied and the nature or number of discrete elements or positions may be altered or varied. Accordingly, all such modifications are intended to be included within the scope of the present disclosure. The order or sequence of any process or method steps may be varied or re-sequenced according to alternative embodiments. Other substitutions, modifications, changes, and omissions may be made in the design, operating conditions and arrangement of the exemplary embodiments without departing from the scope of the present disclosure.

As utilized herein, the terms "approximately," "about," "substantially," and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and are considered to be within the scope of the disclosure.

## Claims

1. A surgical robotics system, comprising:
a robotic arm;
a first cutting tool;
a second cutting tool; and
a computer programmed to:
obtain a planned pose of an implant augment relative to a bone, the implant augment having a curved side and a flat side;
determine whether the curved side or the flat side of the implant augment faces the bone in the planned pose;
in response to a determination that the curved side faces the bone, control the robotic arm to guide the first cutting tool to prepare the bone to receive the implant augment; and
in response to a determination that the flat side faces the bone, control the robotic arm to guide the second cutting tool to prepare the bone to receive the implant augment.

2. The surgical robotics system of claim 1, wherein the second cutting tool is a planer.

3. The surgical robotics system of claim 2, wherein the computer is programmed to control the robotic arm to guide the guide the second cutting tool by defining a virtual cylinder aligned with the planned pose and controlling the robotic arm to constrain the planer to within the virtual cylinder.

4. The surgical robotics system of claim 2, wherein the first cutting tool is a reamer, and wherein the planer and the reamer are selectively attachable to a shaft held by the robotic arm, the surgical robotics system further comprising a power tool operate to provide torque via the shaft to the planer when the planer is attached to the shaft and to the reamer when the reamer is attached to the shaft.

5. The surgical robotics system of any preceding claim, wherein the computer is further programmed to control, in response to the determination that the flat side faces the bone, the robotic arm to guide the first cutting tool to prepare the bone to receive an acetabular cup in a reaming stage.

6. The surgical robotics system of claim 5, wherein the computer is further programmed to instruct, in response to completion of the reaming stage, a user to detach the first cutting tool from the robotic arm and attach the second cutting tool to the robotic arm.

7. The surgical robotics system of any preceding claim, wherein the computer is programmed to determine whether the curved side or the flat side faces the bone by ascertaining an angle of the implant augment in the planned pose relative to a coordinate system defined based on landmarks of the bone and comparing the angle to a range or threshold.

8. The surgical robotics system of any preceding claim, wherein the computer is programmed to determine whether the curved side or the flat side faces the bone by projecting a ray from the flat side and checking whether the ray intersects a virtual model of the bone.

9. The surgical robotics system of any preceding claim, wherein the computer programmed to obtain the planned pose of the implant augment relative to the bone by providing a virtual environment comprising virtual models of the implant augment, the bone, and an implant and enabling a user to adjust relative positions of the virtual models.

10. The surgical robotics system of claim 9, wherein the computer is programmed to enable the user to adjust relative positions of the virtual models by rotating a virtual acetabular cup model about a central axis thereof, and wherein the computer is further programmed to enable placement of virtual screws in the virtual environment.

11. The surgical robotics system of claim 9 or 10, comprising a display screen, wherein the computer is programmed to cause the virtual environment to be presented on the display screen via a graphical user interface, wherein the graphical user interface further comprises a button selectable to flip over the virtual model of the implant augment.

12. A method of configuring a surgical robotics system, comprising:
obtaining a planned pose of an implant augment relative to a bone, the implant augment having a curved side and a flat side;
automatically making a determination as to whether the curved side or the flat side faces the bone in the planned pose;
automatically selecting between cutting tools based on the determination as to whether the curved side or the flat side faces the bone; and
coupling the selected cutting tool to the robotic arm.

13. The method of claim 12, wherein the cutting tools comprise a reamer and a planer.

14. The method of claim 12 or 13, wherein:
in response to the determination being that the flat side faces the bone, a planar is automatically selected as the selected cutting tool; and/or
in response to the determination being that the curved side faces the bone, a reamer is automatically selected as the selected cutting tool.

15. The method of any of claims 12 to 14, wherein automatically making the determination as to whether the curved side or the flat side faces the bone comprises:
ascertaining, by a computer, an angle of the implant augment in the planned pose in a coordinate system, the coordinate system based on landmarks of the bone; and
comparing, by the computer, the angle to a range or threshold.

16. The method of any of claims 12 to 15, wherein obtaining the planned pose of the implant augment relative to the bone comprises providing a graphical user interface enabling a user to adjust the planned pose by moving a virtual augment model relative to a virtual bone model, and wherein moving the virtual augment model comprises flipping the virtual augment model upside down.
